# EUROPEAN PATENT SPECIFICATION

(11) **EP 1 576 919 B1**
(45) Date of publication and mention of the grant of the patent: **30.04.2014**
(21) Application number: 05005736.3
(22) Date of filing: 16.03.2005
(51) Int. Cl.: A61B 1/018, A61B 17/00

(54) **Endoscopic treatment instrument system**
Endoskopisches Behandlungsgerät
Instrument de traitement endoscopique

(30) Priority: 16.03.2004 JP 2004074200
(43) Date of publication of application: 21.09.2005
(73) Proprietor: Olympus Corporation, Shibuya-ku, Tokyo (JP)
(72) Inventor: Tsutomu, Okada, Tachikawa-shi Tokyo (JP)
(74) Representative: von Hellfeld, Axel

(56) References cited:
- EP-A- 1 155 776
- EP-A1- 1 709 900
- US-A- 5 349 940
- US-A- 5 782 748
- US-A- 6 059 719
- PATENT ABSTRACTS OF JAPAN vol. 018, no. 285 (C-1206), 31 May 1994 (1994-05-31) & JP 06 054801 A (OLYMPUS OPTICAL CO LTD), 1 March 1994 (1994-03-01)

## Description

### BACKGROUND OF THE INVENTION

### 1. Field of the Invention

The present invention relates to an endoscopic treatment instrument system.

### 2. Description of the Related Art

When administering a treatment, e.g., a biopsy or the like, to a required part within the body while operating from outside the body by using an endoscope, oftentimes a plurality of treatment instruments such as, for example, a needle knife or a bioptome need to be used sequentially. In doing so, conventionally the operation has been conducted by inserting a selected treatment instrument into the body through a channel provided within an endoscope inserting portion, then extracting the treatment instrument outside the body after conducting a predetermined treatment, and subsequently inserting another treatment instrument into the channel, and so on.

Since these types of treatment instruments have lengths equal to or longer than the endoscope inserting portion, it is necessary to insert a long treatment instrument carefully into the narrow channel from a forceps insertion opening provided in the endoscope at the time of insertion. Therefore, the insertion of the treatment instrument requires high levels of concentration and takes a lot of time and care.

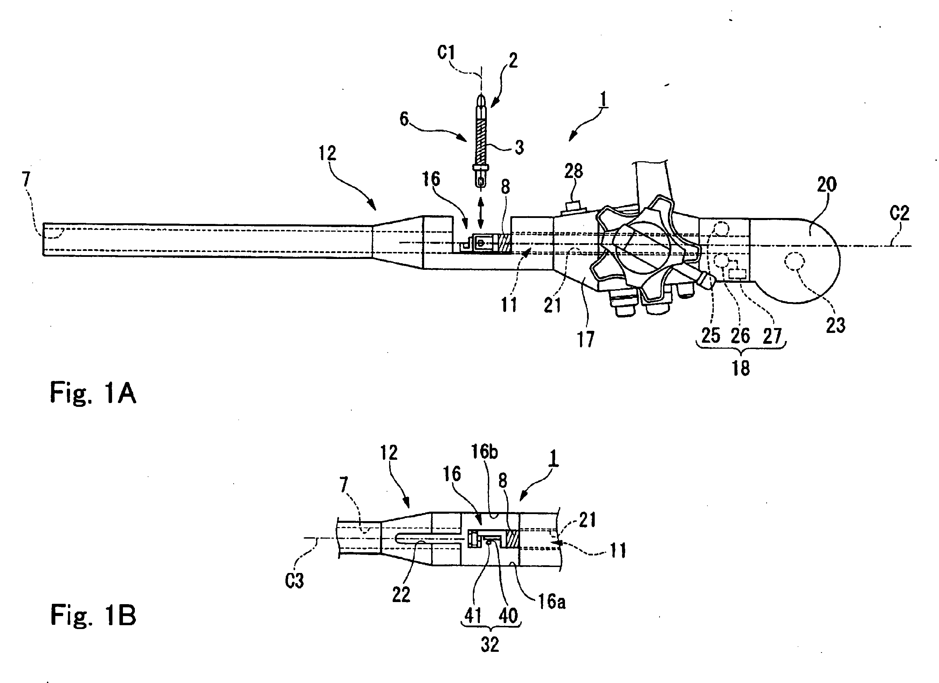

Japanese Laid-Open Patent Publication (Kokai) No. Sho 57-117823 (1982) has suggested an endoscope equipped with an insertion/extraction device for automatically inserting or extracting a treatment instrument to or from a channel shown in FIG. 1 thereof.

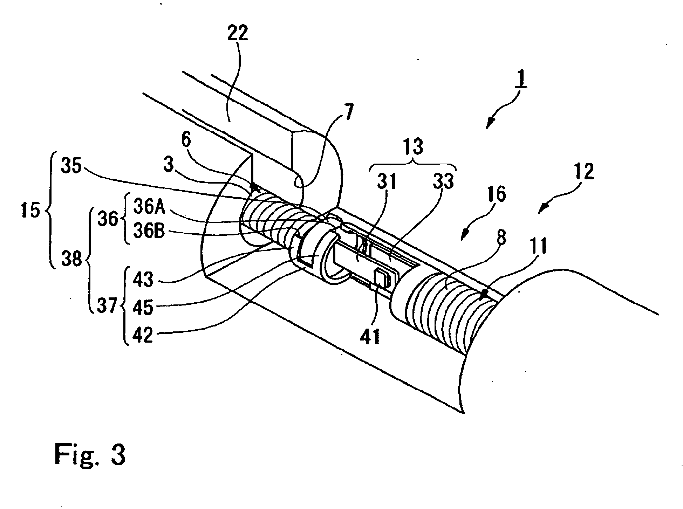

Another Japanese Laid-Open Patent Publication, (Kokai) No. Hei 6-54801 (1994), has suggested an endoscope equipped with a multifunctional instrument shown in FIG. 3 thereof. This endoscope has a plurality of treatment instrument heads. It is used by conveying a selected one of the treatment instrument heads to a distal end of an endoscope inserting portion and connecting it to a distal end of an arm provided inside the inserting portion.

The technology described in Japanese Laid-Open Patent Publication (Kokai) No. Sho 57-117823, however, requires an assistant's help while supporting the treatment instrument with a hand when inserting it into the endoscope, since a conventional treatment instrument is directly used. This results in insufficient time savings and no simplification of any surgical procedure.

In the technology described in Japanese Laid-Open Patent Publication (Kokai) No. Hei 6-54801, the distal end of the arm extending toward the outside of the distal end of the endoscope inserting portion is bent at an angle of 180 degrees and the treatment instrument head is conveyed to the portion and attached/detached by means of an electromagnet. Therefore, unless a sufficient area for this work is secured at the distal end of the endoscope inserting portion, the replacement work cannot be readily performed.

US-A-6059719 discloses an endoscope system comprising a plurality of endoscope modules having different treatment instruments mounted therein. The plurality of endoscope modules having the different treatment instruments mounted therein are freely exchangeable to be coupled to the distal part of the insertion unit of the endoscope. An endoscopic treatment system according to the preamble of claim 1 is disclosed in US-A-5782748.

It is an object of the present invention to provide an endoscopic treatment instrument system that facilitates replacement of a treatment instrument and enables saving time and simplifying the surgical procedure.

### BRIEF SUMMARY OF THE INVENTION

An endoscopic treatment instrument system according to the present invention is described in claim 1. The dependent claims describe preferred embodiments of that system.

With this arrangement, the treatment instrument head can move within the channel of the endoscope while being attached to the treatment instrument driving mechanism. Therefore, the freedom in selecting a place for replacing the treatment instrument head is increased. For example, the endoscopic treatment instrument system can have a place, i.e., a location, for replacing the treatment instrument head at or in the vicinity of an opening on the side of a proximal end of the channel of the endoscope, where the operator's maneuverability and ability to change a head is far easier. Generally an operating portion or the like is provided on the side of the proximal end of the endoscope. Therefore, replacement of the treatment instrument head on the side of the proximal end leads to an increase in workability, efficiency and ease of use.

Preferably, the endoscopic treatment instrument system of the present invention has a first engagement mechanism for allowing the treatment instrument driving mechanism and the treatment instrument head to be coupled together when their central axes intersect, and further allows them to become engaged when their central axes align with each other. The first engagement mechanism serves to bring about the engagement between a proximal end of the first sheath tube portion and a distal end of the second sheath tube portion or between a proximal end of the first operating member and a distal end of the second operating member.

With this arrangement, the engagement between both units can be easily achieved by tilting the treatment instrument head relative to the treatment instrument driving mechanism to fit them together, followed by orienting the treatment instrument head and the treatment instrument driving mechanism in the same direction.

Preferably, the endoscopic treatment instrument system of the present invention has a second engagement mechanism for engaging the treatment driving mechanism and the instrument head when their central axes coincide, through rotational movement between them using the first engagement mechanism as a center of the turning action. The second engagement mechanism serves to bring about engagement between the proximal end of the first sheath tube portion and the distal end of the second sheath tube portion or between the proximal end of the first operating member and the distal end of the second operating member as the other engagement not conducted by the first engagement mechanism.

With this arrangement, the engagement of the second engagement mechanism can be achieved in conjunction with the engagement action of the first engagement mechanism. More specifically, the turning action involving directing the treatment instrument head and the treatment instrument driving mechanism in the same direction of the first engagement mechanism serves as the engagement action of the second engagement mechanism. This improves the endoscope's operation.

A swinging member which supports and turns the treatment instrument head stabilizes the turning action. Preferably, the endoscopic treatment instrument system further comprises a restricting member, i.e., a stop, for restricting the turning action of the swinging member when the central axis of the treatment instrument driving mechanism and that of the treatment instrument head coincide with each other.

Furthermore, the treatment instrument head may be accommodated in an accommodating portion (for example, a box) and the swinging member may be provided in the accommodating portion. The accommodating portion is preferably positioned and fixed at the proximal end of the channel of the endoscope.

An example of the first engagement mechanism includes a first connecting member connected to the proximal end of the first operating member and having a through hole longer in an axial direction of the treatment instrument head, and a second connecting member connected to the distal end of the second operating member and having a projection which is longer in a direction perpendicular to an axial direction of the treatment instrument driving mechanism and which can fit into the through hole.

Another example of the first engagement mechanism includes a first connecting member connected to the proximal end of the first operating member and having a through hole longer in a direction perpendicular to an axial direction of the treatment instrument head and a second connecting member connected to the distal end of the second operating member and having a projection which is longer in an axial direction of the treatment instrument driving mechanism and which can fit into the through hole.

An example of the second engagement mechanism includes an engagement portion provided at the proximal end of the first sheath tube portion, a notch portion, which is provided at the distal end of the second sheath tube portion and into which the engagement portion can be inserted from a turning direction of the treatment instrument head, and a restricting member engageable with the engagement portion and restricting axial movement of the engagement portion.

Another example of the second engagement mechanism includes an engagement portion provided at the proximal end of the first sheath tube portion, a notch portion, which is provided at the distal end of the second sheath tube portion and into which the engagement portion can be inserted from a protruding side of the projection, and a restricting member restricting movement in the axial direction of the engagement portion.

According to the present invention, an operator can handle a plurality of treatment instruments using a single treatment instrument driving mechanism by preparing a plurality of treatment instrument heads and replacing them one after another, thereby avoiding the burden of having to handle long treatment instruments for each treatment step. This facilitates replacing of treatment instruments, and reduces the time needed for and generally simplifies surgical procedures.

### BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:
FIGS. 1A and 1B show an endoscopic treatment instrument system according to a first embodiment of the present invention: FIG. 1A is a side view and FIG. 1B is a partial front view;
FIG. 2 is a perspective view showing a relevant part of the endoscopic treatment instrument system according to the first embodiment of the present invention;
FIG. 3 is an explanatory diagram showing a condition where a treatment instrument head is connected to a treatment instrument driving mechanism in the endoscopic treatment instrument system according to the first embodiment of the present invention;
FIG. 4 is a perspective view showing a relevant part of the endoscopic treatment instrument system according to a second embodiment of the present invention;
FIG. 5 is an explanatory diagram showing a condition where a treatment instrument head is connected to a treatment instrument driving mechanism in the endoscopic treatment instrument system according to the second embodiment of the present invention;
FIGS. 6A, 6B, and 6C show an endoscopic treatment instrument system according to a third embodiment of the present invention: FIG. 6A is a side view, FIG. 6B is a partial front view, and FIG. 6C is a partially enlarged view;
FIG. 7 is a perspective view showing a relevant part of the endoscopic treatment instrument system according to the third embodiment of the present invention;
FIG. 8 is an explanatory diagram showing a condition where a treatment instrument head is being engaged with a treatment instrument driving mechanism in the endoscopic treatment instrument system according to the third embodiment of the present invention;
FIG. 9 is an explanatory diagram showing a condition where the treatment instrument head has been engaged with the treatment instrument driving mechanism in the endoscopic treatment instrument system according to the third embodiment of the present invention;
FIG. 10 is an explanatory diagram showing a condition where the treatment instrument head is connected to the treatment instrument driving mechanism in the endoscopic treatment instrument system according to the third embodiment of the present invention;
FIG. 11 is a perspective view showing an accommodating portion of an endoscopic treatment instrument system according to a fourth embodiment of the present invention;
FIG. 12 is a rear view showing the accommodating portion of the endoscopic treatment instrument system according to the fourth embodiment of the present invention;
FIGS. 13A and 13B show an endoscope of an endoscopic treatment instrument system according to the fourth embodiment of the present invention: FIG. 13A is a side view and FIG. 13B is a relevant-part enlarged view.
FIG. 14 is a perspective view showing the endoscopic treatment instrument system according to the fourth embodiment of the present invention;
FIGS. 15A and 15B are explanatory diagrams each showing a condition where a treatment instrument head is connected to a treatment instrument driving mechanism in the endoscopic treatment instrument system according to the fourth embodiment of the present invention;
FIG. 16 is an explanatory diagram showing a condition where the treatment instrument head is connected to the treatment instrument driving mechanism in the endoscopic treatment instrument system according to the fourth embodiment of the present invention;
FIG. 17 is a perspective view showing an endoscopic treatment instrument system according to a fifth embodiment of the present invention;
FIG. 18 is an explanatory diagram showing a condition where a treatment instrument head is connected to a treatment instrument driving mechanism in the endoscopic treatment instrument system according to the fifth embodiment of the present invention;
FIGS. 19A, 19B, 19C, and 19D show an endoscope of the endoscopic treatment instrument system according to the fifth embodiment of the present invention: FIG. 19A is a side view, FIG. 19B is a relevant-part enlarged view, FIG. 19C is a partially enlarged view, and FIG. 19D is a partially enlarged view;
FIG. 20 is a perspective view showing the endoscope of the endoscopic treatment instrument system according to the fifth embodiment of the present invention; and
FIG. 21 is a perspective view showing the endoscopic treatment instrument system according to the fifth embodiment of the present invention.

### DETAILED DESCRIPTION OF THE DRAWINGS

Preferred embodiments of the invention will be described below with reference to the accompanying drawings.

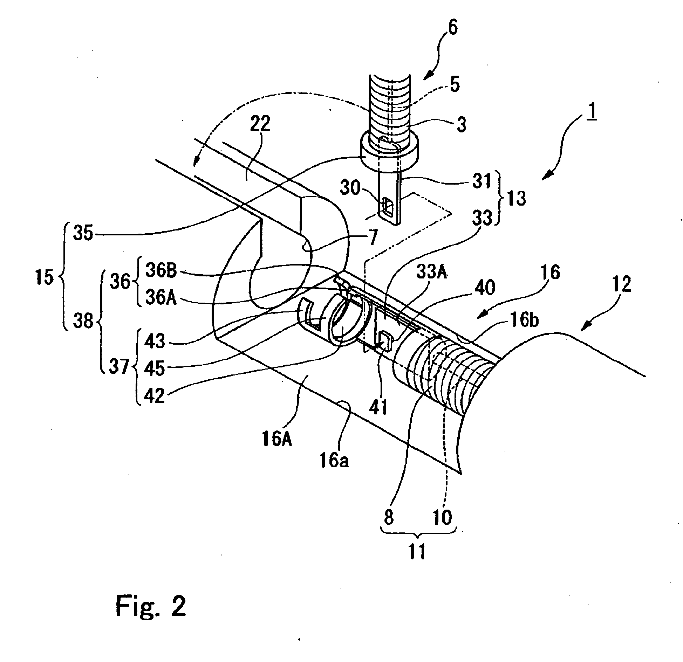

The first embodiment of the present invention will be described with reference to FIG. 1A to FIG. 3.

An endoscopic treatment instrument system 1 according to the first embodiment has a replaceable treatment instrument head 6 and an endoscope 12. The treatment instrument head 6 includes the first flexible sheath tube portion 3, a treatment portion 2 supported by the first sheath tube portion 3 at a distal end and suitable for conducting a treatment responsive to an external operation. A first operating wire (the first operating member) 5 is retractably arranged within the first sheath tube portion 3 serves to drive the treatment portion 2.

The endoscope 12 has a channel 7 through which a treatment instrument head 6 can pass and an operating portion 17. The endoscope 12 further has a treatment instrument driving mechanism 11 including the second sheath tube portion 8 which is retractable within the channel 7. The second operating wire (the second operating member) 10 is retractable within the second sheath tube portion 8.

Further, the endoscopic treatment instrument system has the first engagement mechanism 13 and the second engagement mechanism 15 in order to engage the treatment instrument head 6 with the treatment instrument driving mechanism 11. In the first embodiment, the first engagement mechanism 13 is used for engaging the first operating wire 5 of the treatment instrument head 6 with the second operating wire 10 of the treatment instrument driving mechanism 11, and the second engagement mechanism 15 is used for engaging the first sheath tube portion 3 of the treatment instrument head 6 with the second sheath tube portion 8 of the treatment instrument driving mechanism 11.

The first engagement mechanism 13 allows a proximal end of the first operating wire 5 of the treatment instrument head 6 to be coupled to a distal end of the second operating wire 10 of the treatment instrument driving mechanism 11 when a central axis C1 of the treatment instrument head 6 is substantially perpendicular to a central axis C2 of the treatment instrument driving mechanism 11. The wires engage when their central axes are then aligned with each other.

The second engagement mechanism 15 makes a proximal end of the first sheath tube portion 3 of the treatment instrument head 6 engage with a distal end of the second sheath tube portion 8 of the treatment instrument driving mechanism 11 when the central axis of the treatment instrument driving mechanism 11 and that of the treatment instrument head 6 are aligned with each other, with the first engagement mechanism 13 serving as a center of the turning action.

The endoscope 12 is provided with an instrument insertion opening 16 for use in attaching or detaching the treatment instrument head 6 to or from the treatment instrument driving mechanism 11 at a proximal end of the channel 7.

The endoscope 12 has an insertion/extraction mechanism 18 for inserting or extracting the treatment instrument driving mechanism 11 into or from the channel 7 arranged at a proximal end of the operating portion 17 of the endoscope 12. Furthermore, it has an accommodation mechanism 20, e.g., a storage space, for accommodating the treatment instrument driving mechanism 11 arranged at a proximal end of the insertion/extraction mechanism 18.

In the operating portion 17, there is arranged a through path 21, which provides special communication between the instrument insertion opening 16 and the accommodation mechanism 20 and which the treatment instrument driving mechanism 11 can pass. Furthermore, there is provided a slit 22 into which the treatment instrument head 6 can be inserted from a radial direction of the operating portion 17, the slit being provided along a central axis of the channel 7 and extending from the instrument insertion opening 16 toward a distal end of the channel 7.

The accommodation mechanism 20 is provided with a winding member 23 that can wind the treatment instrument driving mechanism 11 on its outer peripheral surface.

The insertion/extraction mechanism 18 includes a pair of rollers 25 and 26 juxtaposed to each other and holding the treatment instrument driving mechanism 11 therebetween. A driving portion 27 is coupled to and selectively rotates the roller 26, in opposite directions, responsive to a control signal from a switch 28 arranged in the operating portion 17.

The first engagement mechanism 13 has the first connecting member 31 on the side of the treatment instrument head 6 and the second connecting member 33 on the side of the treatment instrument driving mechanism 11. The first connecting member 31, which is formed of a strip of thin plate, is connected to the proximal end of the first operating wire 5, having an axially wider through hole 30 in the end portion. The second connecting member 33, has a substantially a semicylindrical shape along the central axis C2, and is connected to the distal end of the second operating wire 10, its projection 32 protruding from a plane portion 33A laying in the central axis C2 and filling into the through hole 30.

At the time of engagement of the first engagement mechanism 13, the first connecting member 31 is placed in such a way that the direction of the plate thickness thereof is aligned with the direction of the width of the slit 22. Furthermore, the plane portion 33A of the second connecting member 33 is placed facing toward one end 16a of a wall 16A along the slit 22. The projection 32 has a supporting shaft 40 and a head 41. The supporting shaft 40 is arranged standing taller than the plane thickness of the first connecting member 31 and formed with a diameter smaller than the through hole 30. The head 41 is arranged at a distal end of the supporting shaft 40 with the direction of the longer side coincident with the width direction of the second connecting member 33. The head 41 is able to protrude via the through hole 30 of the first connecting member 31 with the central axis C1 of the treatment instrument head 6 perpendicular to the central axis C2 of the treatment instrument driving mechanism 11.

Further, the second engagement mechanism 15 has a round flange 35 on the side of the treatment instrument head 6 and a support 38 on the side of the treatment instrument driving mechanism 11. The flange 35 is arranged at the proximal end of the first sheath tube portion 3. The support 38, which is arranged at the distal end of the second sheath tube portion 8, includes a notch portion 36 into which the flange 35 can be inserted from the turning direction of the treatment instrument head 6 and a restricting member 37 that engages the flange 35 and restricts its axial movement.

The restricting member 37 has two ring members 43 and 45 connected via a connecting portion 42, and spaced a width of the flange 35 away from each other.

The first sheath tube portion 3 and the second sheath tube portion 8 are each formed of a flexible coil.

The notch portion 36 has a first notch portion 36A and a second notch portion 36B. The first notch portion 36A is formed with a cut in a portion of the ring member 45 corresponding to the opening direction of the instrument insertion opening 16, for permitting insertion of the first connecting member 31. The second notch portion 36B has a cut in a portion of the ring member 43 corresponding to the opening direction of the instrument insertion opening 16, for permitting insertion of the first sheath tube portion 3. The connecting portion 42 connects the outer peripheral surfaces of the ring members 43 and 45, which are opposed to the notch portion 36. The connecting portion outer surface is curved to complement the outer peripheral surface of the flange 35 and recessed from the inner peripheral surfaces of the ring members 43 and 45.

The operating method of the endoscopic treatment instrument system 1 according to this embodiment is as follows.

First, the operator drives the driving portion 27 by activating the switch 28 to rotate the rollers 25 and 26 in a direction which advances the treatment instrument driving mechanism 11 toward the distal end of the channel 7 and advances the treatment instrument driving mechanism 11 wound on the winding member 23 toward the channel 7.

Subsequently, the operator stops the driving portion 27 with the switch 28 when the distal end of the treatment instrument driving mechanism 11 has advanced from inside of the through path 21 to the instrument insertion opening 16. Then, the operator inserts the treatment instrument head 6 into the instrument insertion opening 16 from the side of the first connecting member 31. At the time of insertion, the central axis C1 of the treatment instrument head 6 is oriented so that it is substantially perpendicular to the central axis C2 of the treatment instrument driving mechanism 11. The through hole 30 and the head 41 of the projection 32 are directed with their longer directions coincident with each other, so that the through hole 30 of the first connecting member 31 can fit on the head 41 of the second connecting member 33. Then, the operator inserts the head 41 through the through hole 30. Thereafter, the treatment instrument head 6 is turned around the supporting shaft 40 at about 90 degrees, until the central axis C1 of the treatment instrument head 6 becomes aligned with the central axis C2 of the treatment instrument driving mechanism 11 and its body is inserted into the channel 7 via the slit 22.

When the central shaft C1 is aligned with the central axis C2, the direction of the longer side of the through hole 30 is substantially perpendicular to that of the head 41, and the first connecting member 31 and the second connecting member 33 are then securely coupled to each other.

At this point, the first connecting member 31 and the proximal end of the first sheath tube portion 3 are inserted into the first notch portion 36A and the second notch portion 36B, respectively, and the flange 35 is inserted between the ring members 43 and 45 at the same time, thereby achieving engagement with the support 38.

Thus, the treatment instrument head 6 is connected to the treatment instrument driving mechanism 11 with their relative displacement axially restricted.

Subsequently, the operator drives the driving portion 27 by operating the switch 28 again to move the treatment instrument driving mechanism 11 toward the distal end of the channel 7, where the treatment instrument head 6 protrudes from the channel 7 and is able to carry out a given treatment.

After completing a treatment, the operator moves the treatment instrument head 6 to the instrument insertion opening 16 while leaving the winding member 23 to wind the treatment instrument driving mechanism 11. Then, he or she takes out the treatment instrument head 6 from the slit 22 and turns it around the supporting shaft 40 in the direction in which the central axis C1 is perpendicular to the central axis C2, thereby releasing the hold at the first engagement mechanism 13 and at the second engagement mechanism 15. The operator then detaches and removes the treatment instrument head 6 from the treatment instrument driving mechanism 11.

With the endoscopic treatment instrument system 1, the treatment instrument head 6 can be readily attached to or detached from the treatment instrument driving mechanism 11 in the instrument insertion opening 16 of the operating portion 17. Thereby, the operator can work while checking the attachment or detachment operation at hand, and with a simple operation of turning the treatment instrument head 6, can easily attach it or detach it from the treatment instrument driving mechanism 11. Therefore, the operator can easily replace the treatment instrument and easily conduct a surgical procedure in a shorter time period.

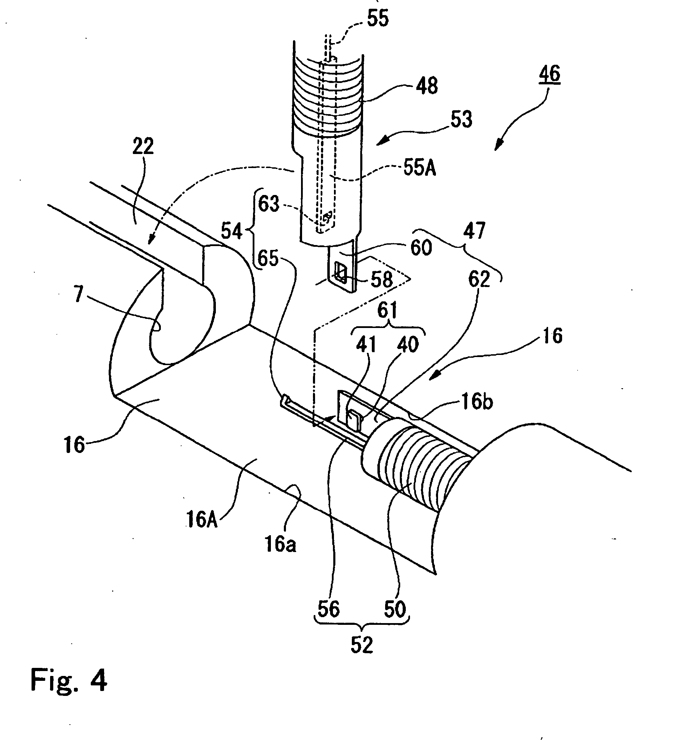

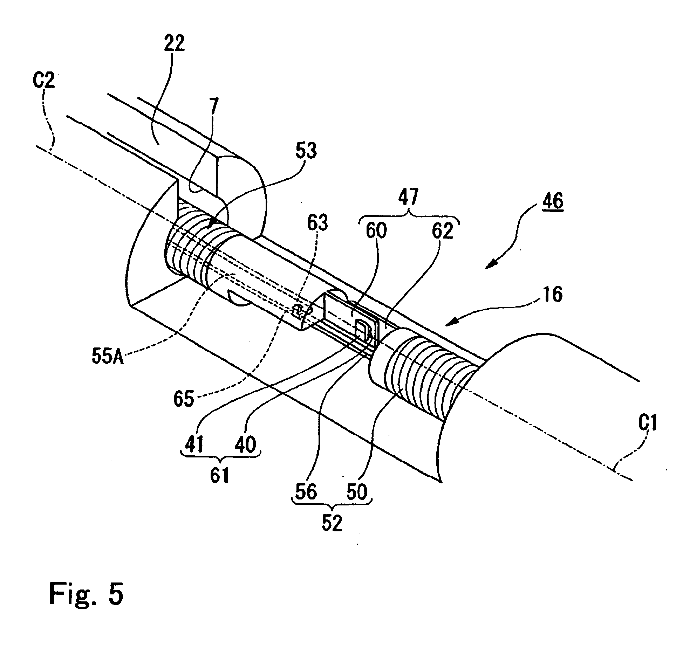

The following describes the second embodiment with reference to FIGS. 4 and 5.

The same reference numerals have been used as in FIG. 1 for similar parts and their description is therefore omitted.

The main difference between the second embodiment and the first embodiment is in the manner of engagement between the first engagement mechanism and the second engagement mechanism. In the endoscopic treatment instrument system 1 according to the first embodiment, the first engagement mechanism 13 engages the proximal end of the first operating wire 5 with the distal end of the second operating wire 10 and the second engagement mechanism 15 engages the proximal end of the first sheath tube portion 3 with the distal end of the second sheath tube portion 8. But with the endoscopic treatment instrument system 46 according to the second embodiment, the first engagement mechanism 47 allows the proximal end of the first sheath tube portion 48 to fit on the proximal end of the second sheath tube portion 50 when the central axis of a treatment instrument driving mechanism 52 is substantially perpendicular to that of a treatment instrument head 53, and makes them engage with each other when the central axes become aligned with each other. Moreover, in the second embodiment, the second engagement mechanism 54 makes the proximal end of the first operating wire 55 engage with the distal end of the second operating wire 56 when the central axis of the treatment instrument driving mechanism 52 is pivoted to align with the central axis of the treatment instrument head 53, with the first engagement mechanism 47 serving as the pivot point of the pivoting action.

The first engagement mechanism 47 includes the first connecting member 60, which is connected to a proximal end of the first sheath tube portion 48 and has a first through hole 58 wider in the axial direction. The second connecting member 62, which is connected to a distal end of the second sheath tube portion 50 and has a first projection 61 protruding to fit into the first through hole 58. The first projection 61 is connected to a head 41, which is wider in a direction perpendicular to the central axis C2 of the treatment instrument driving mechanism 52.

The second engagement mechanism 54 includes the second through hole 63 arranged at a proximal end of the plate-shaped, connecting member 55A connected to a proximal end of the first operating wire 55. The second projection 65 provided at a distal end of the second operating wire 56, by being bent into an opening direction of an instrument insertion opening 16.

The following describes an operating method of the endoscopic treatment instrument system 46 according to the second embodiment.

First, similarly to the first embodiment, an operator advances a distal end of the treatment instrument driving mechanism 52 to the instrument insertion opening 16.

Subsequently, the operator inserts the treatment instrument head 53 into the instrument insertion opening 16 from the side of the first connecting member 60. Then, similarly to the first embodiment, the operator fits the head 41 of the second connecting member 62 through the hole 58 of the first connecting member 60. The operator then turns the treatment instrument head 53 about 90 degrees around a supporting shaft 40 in a direction in which the central axis C1 of the treatment instrument head 53 becomes aligned with the central axis C2 of the treatment instrument driving mechanism 52 and inserts it into a channel 7 from a slit 22. The first through hole 58 and the head 41 are then oriented with their longer directions substantially perpendicular to each other, by which the first connecting member 60 securely engages with the second connecting member 62.

At this moment, the second projection 65 is inserted into the second through hole 63, by which the first operating wire 55 engages with the second operating wire 56.

The endoscopic treatment instrument system 46 provides the same action and effect as the first embodiment.

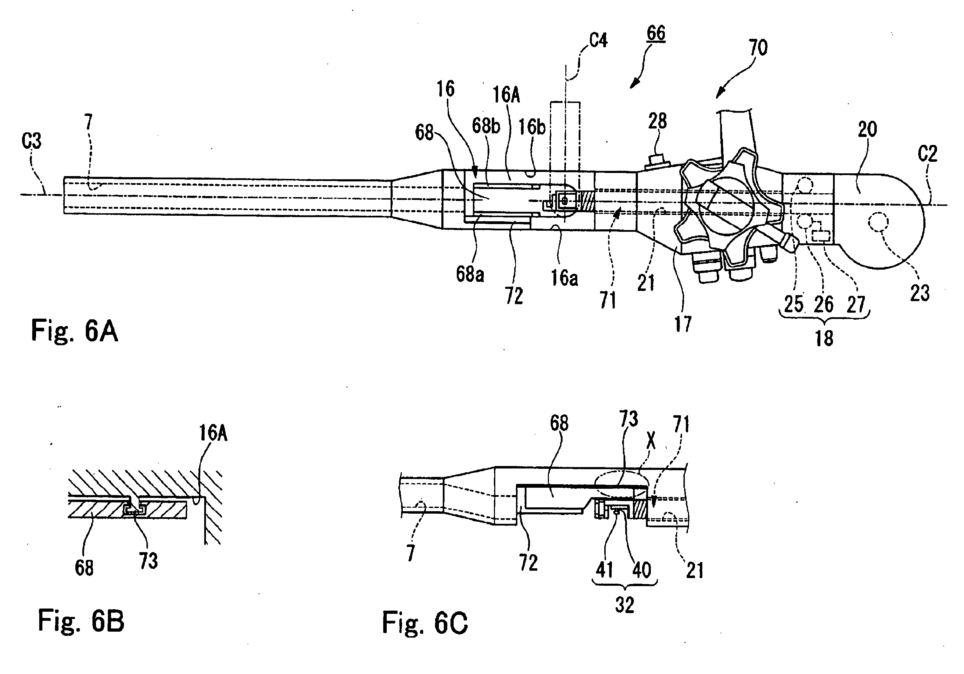

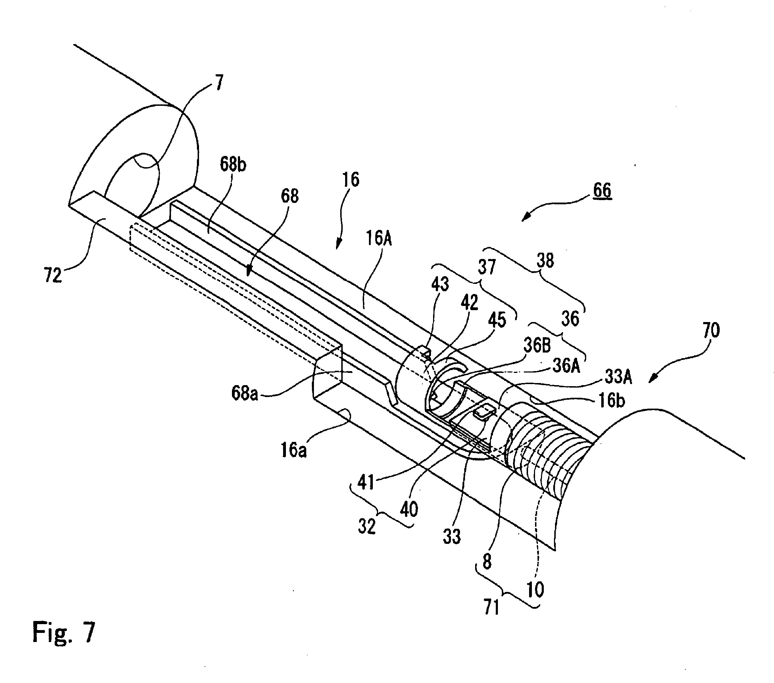

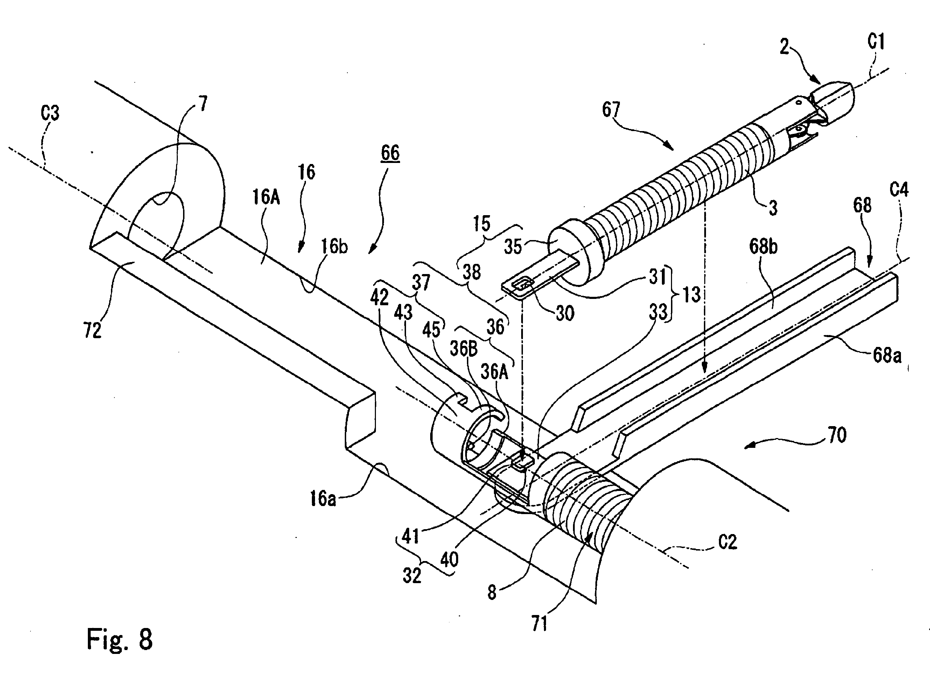

The following describes the third embodiment with reference to FIGS. 6A to 10.

The same reference numerals have been used as in the foregoing other embodiments for similar parts and their description is therefore omitted.

The difference between the third embodiment and the first embodiment is that an endoscopic treatment instrument system 66 according to this embodiment has a swinging member 68, which is rotatable while supporting a treatment instrument head 67 around the first engagement mechanism 13, arranged in an endoscope 70. Furthermore, the endoscope 70 has a restricting member (restricting means) 72, i.e., a stopper, which restricts rotation of the swinging member 68 once the central axis C1 of the treatment instrument head 67 is coincident with the central axis C2 of the treatment instrument driving mechanism 71.

An instrument insertion opening 16 is formed by removing a part of a channel 7 substantially along the entire cross section of the channel 7 which is exposed to the outside.

The swinging member 68 is formed with a width and a depth permitting accommodation of the treatment instrument head 67, with a substantially U shape cross section open on the opening side of the instrument insertion opening 16. The swinging member 68 is rotatably connected at its end to a turning shaft 73 protruding from a wall portion 16A of the instrument insertion opening 16 on a central axis C3 of the channel 7. The swinging member 68 is formed with a length which can accommodate within the instrument insertion opening 16 the treatment instrument head 67. The side face corresponding to the side of one end 16a of the instrument insertion opening 16 of the swinging member 68 is referred to here as the one-end side face 68a, and the side face corresponding to the other end 16b as the other-end side face 68b.

The plate-shaped restricting member 72 is arranged in a standing condition with a height permitting its abutment against the one-end side face 68a of the swinging member 68 on the side of one end 16a of the wall portion 16A along the channel 7.

The first connecting member 31 is arranged in a direction in which the direction of the plate width is parallel to the wall portion 16A. A plane portion 33A of the second connecting member 33 is arranged facing toward the opening portion of the instrument insertion opening 16, with a supporting shaft 40 arranged to protrude from the plane portion 33A.

The first notch portion 36A and the second notch portion 36B of the second connecting member 33 are opened toward the side of the other end 16b of the wall portion 16A.

The following describes an operating method of the endoscopic treatment instrument system 66 according to this embodiment and the actions and effects thereof.

First, an operator operates an insertion/extraction mechanism 18 and moves the treatment instrument driving mechanism 71 to the position where the projection 32 matches the turning shaft 73. The operator then rotates the swinging member 68 around the turning shaft 73 in the direction in which it becomes spaced from the restricting member 72, to the position where the central axis C4 of the swinging member 68 is substantially perpendicular to the central axis C2 of the treatment instrument driving mechanism 71.

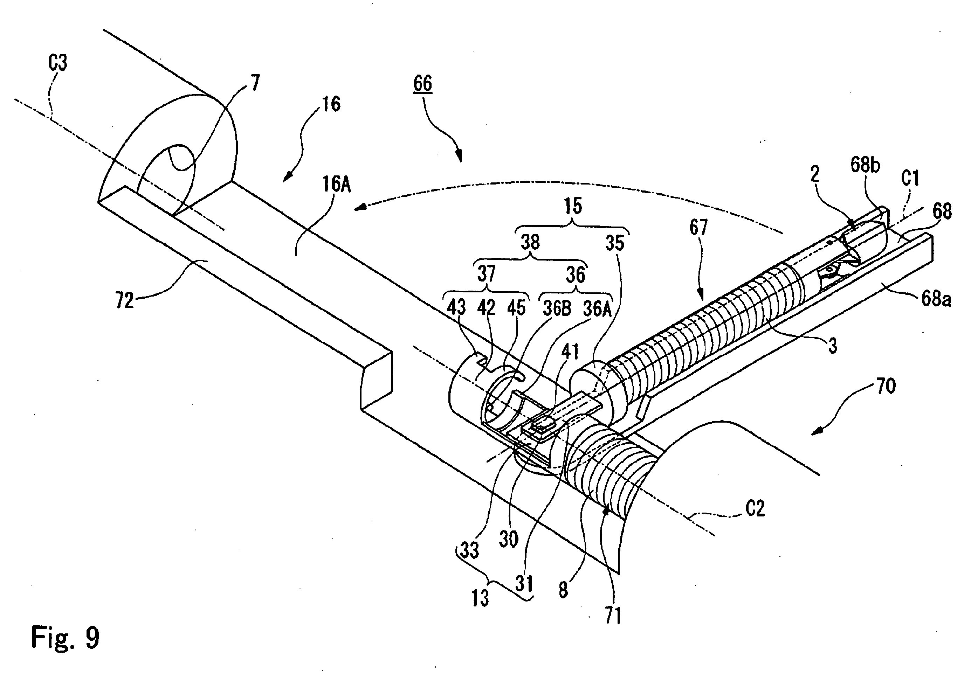

In this condition, as shown in FIG. 9, the operator places the treatment instrument head 67 in the swinging member 68 enclosed by the one-end side face 68a and the other-end side face 68b while fitting the through hole 30 of the first connecting member 31 on the projection 32 of the second connecting member 33. The operator then rotates the swinging member 68 around the turning shaft 73 to the position where the rotation of the swinging member 68 is restricted by the restricting member 72, in a direction in which the central axis C1 of the treatment instrument head 67 eventually aligns with the central axis C2 of the treatment instrument driving mechanism 71, with the through hole 30 reaching the supporting shaft 40.

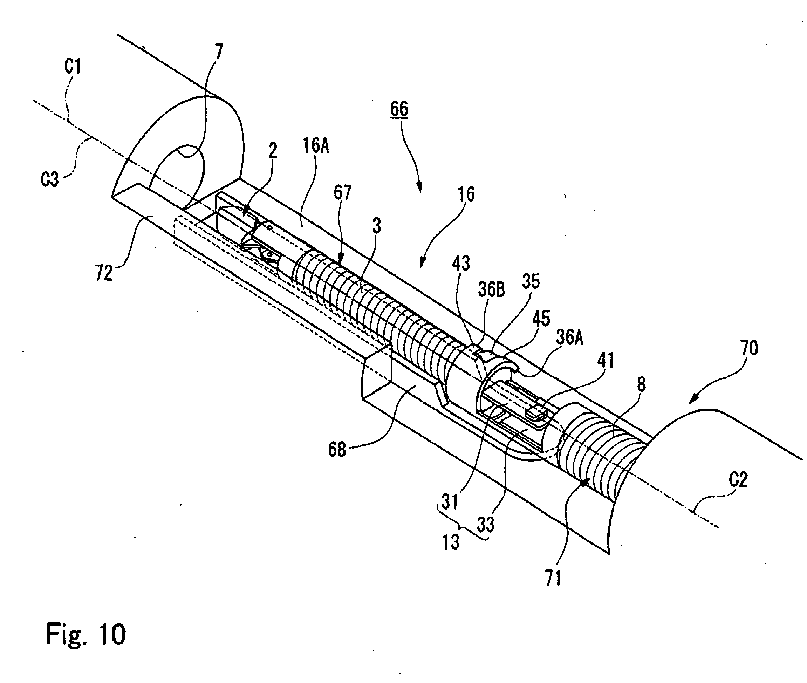

At this point, as shown in FIG. 10, the central axis C1 is coincident with the central axis C2 and the direction of the long side of the through hole 30 is substantially perpendicular to that of the head 41, which locks the first connecting member 31 with the second connecting member 33.

Concurrently, the first connecting member 31 is inserted into the first notch portion 36A and a proximal end of the first sheath tube portion 3 is inserted into the second notch portion 36B, by which a flange 35 is inserted between ring members 43 and 45, thereby achieving engagement with the support 38.

According to this endoscopic treatment instrument system 66, when the treatment instrument head 67 is connected to the treatment instrument driving mechanism 71, the operator rotates the swinging member 68 in the direction in which the central axis of the treatment instrument head 67 becomes aligned with that of the treatment instrument driving mechanism 71, by which the treatment instrument head 67 can be connected to the treatment instrument driving mechanism 71 by means of the first engagement mechanism 13 and the second engagement mechanism 15. This obtains a more reliable connection of the treatment instrument head 67 to the treatment instrument driving mechanism 71.

At this time, the abutment of the swinging member 68 against the restricting member 72 enables the central axis of the treatment instrument head 67 to be aligned with that of the treatment instrument driving mechanism 71 more easily and reliably, thereby achieving a reliable connection between them.

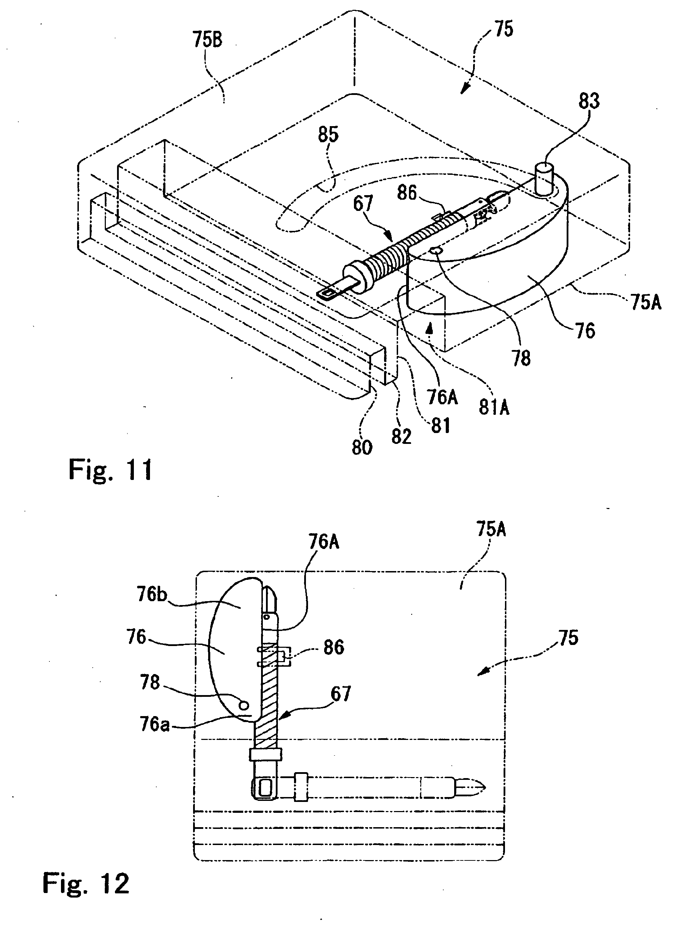

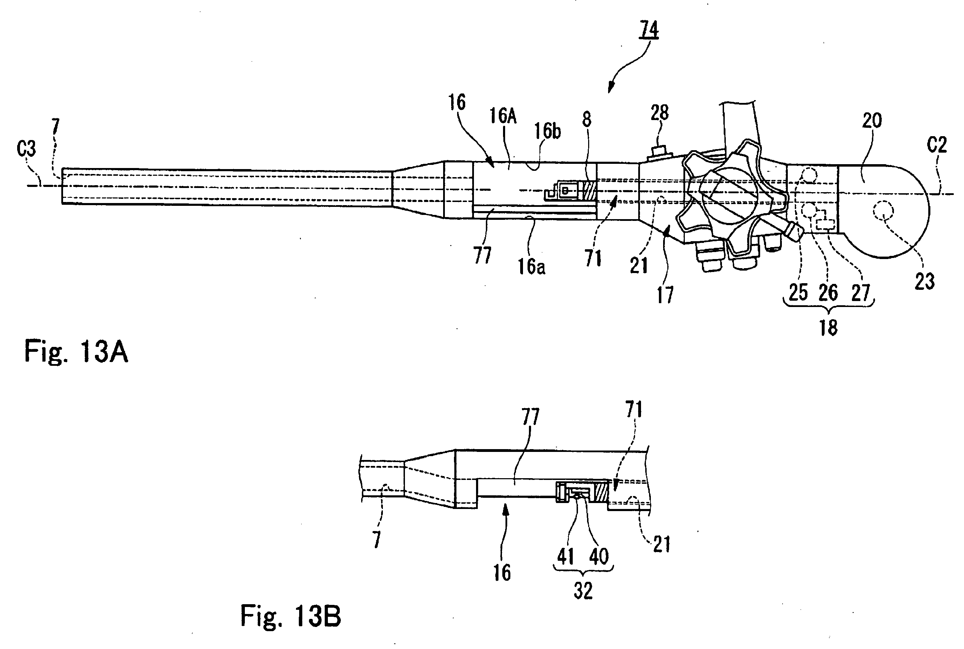

The following describes the fourth embodiment with reference to FIGS. 11 to 16.

Here again, the same reference numerals have been used as in the foregoing other embodiments for similar parts and their description is therefore omitted.

The main difference between the fourth embodiment and the third embodiment is that the endoscopic treatment instrument system 74 according to the fourth embodiment has the treatment instrument head 67 accommodated in a housing box (accommodating portion) 75 as shown in FIGS. 11 to 13B. The housing box 75 also accommodates the swinging member 76. The swinging member 76 is rotatably arranged with the treatment instrument head 67 around a pivot 78 arranged within the housing box 75. A positioning member 77 is arranged on the side of the endoscope. It positions the housing box 75 at the instrument insertion opening 16 in such a way that the swinging of the treatment instrument head 67 accompanying the swinging of the swinging member 76 is made around the first engagement mechanism 13.

The positioning member 77 is plate shaped and arranged in a standing condition, axially extending on the side of one end 16a of the wall portion 16A along the channel 7.

One side of the housing box 75 has a length that enables its accommodation in the instrument insertion opening 16 when the direction of the central axis C3 is assumed to be the longer direction. The housing box 75 is provided with the first groove 80 that can engage the positioning member 77, and a depth that permits engagement with the positioning member 77. The first groove 80 is provided at a bottom surface 75A of the housing box 75 opposed to the wall portion 16A of the instrument insertion opening 16.

In addition, the second groove 81, which the treatment instrument head 67 and the treatment instrument driving mechanism 71 can pass through when the positioning member 77 is inserted into the first groove 80, is arranged in parallel to the first groove 80 at the bottom surface 75A.

A partition 82 is arranged between the second groove 81 and the first groove 80. However, there is no wall at one side of the second groove 81 that faces the side of another edge 16b of the wall portion 16A when the housing box 75 is attached to the positioning member 77, and this side is kept as an opened area 81 for enabling the treatment instrument head 67 to move from the housing box 75 to the inside of the second groove 81.

The swinging member 76 has an abutment surface 76A, enabling the treatment instrument head 67 to move within the housing box 75 with abutting the treatment instrument head 67. The swinging member 76 is connected to the pivot 78 arranged in the vicinity of the second groove 81 at the bottom surface 75A on the side of the one end 76a.

On the side of the other end 76b of the swinging member 76, a gripper 83 protrudes from the housing box 75 permitting turning of the swinging member 76.

A guide slot 85 is formed on an upper surface 75B of the housing box 75. The guide slot 85 enables the operator to turn the swinging member 76 around the pivot 78 from the direction in which the swinging member 76 is perpendicular to the second groove 81 toward the second groove 81 by gripping the gripper 83 with the treatment instrument head 67 accommodated in the housing box 75.

On the bottom surface 75A of the housing box 75, there is provided a latching member 86 that can latch the treatment instrument head 67 between the abutment surface 76A and the latching member 86 when the swinging member 76 is arranged perpendicularly to the second groove 81. The latching member 86 is formed by turning up a part of the bottom surface 75A toward the upper surface 75B.

The following describes an operating method of the endoscopic treatment instrument system 74 according to this embodiment.

First, an operator operates an insertion/extraction mechanism 18 of the endoscope to move the treatment instrument driving mechanism 71 to the position where the central axis of the projection 32 coincides with that of the through hole 30 when the positioning member 77 is inserted into the first groove 80 of the housing box 75.

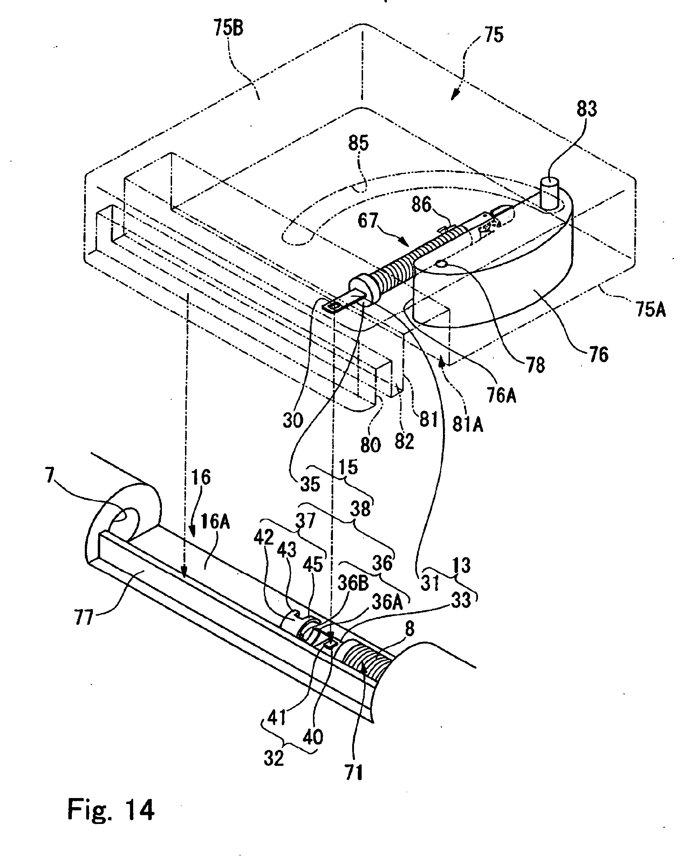

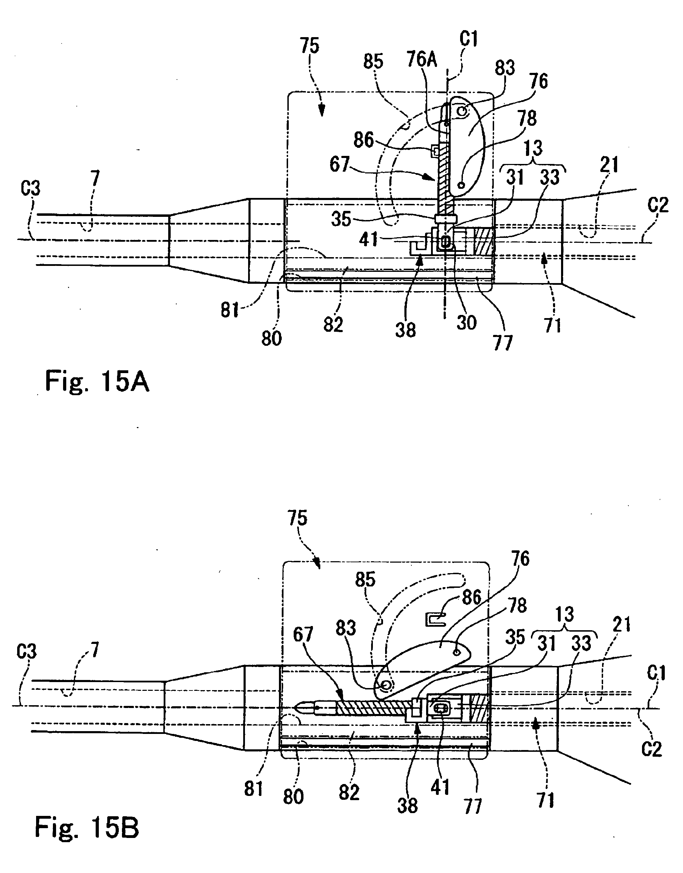

As shown in FIG. 14, when the operator attaches the housing box 75 to the positioning member 77 in this condition, the through hole 30 of the first connecting member 31 fits on the projection 32 of the second connecting member 33. As shown in FIG. 15A, the operator then rotates the swinging member 76 around the pivot 78 by gripping the gripper 83 of the swinging member 76 in a direction which aligns the central axis C1 of the treatment instrument head 67 with the central axis C2 of the treatment instrument driving mechanism 97 with the through hole 30 reaching the supporting shaft 40.

When the central axis C1 is aligned with the central axis C2, the first connecting member 31 engages with the second connecting member 33 with the direction of a longer side of the through hole 30 substantially perpendicular to that of the head 41 as shown in FIG. 15B.

Concurrently, the first connecting member 31 becomes inserted into the first notch portion 36A, the proximal end of the first sheath tube portion 3 inserts into the second notch portion 36B, and the flange 35 inserts between the ring members 43 and 45, thereby engaging with the support 38.

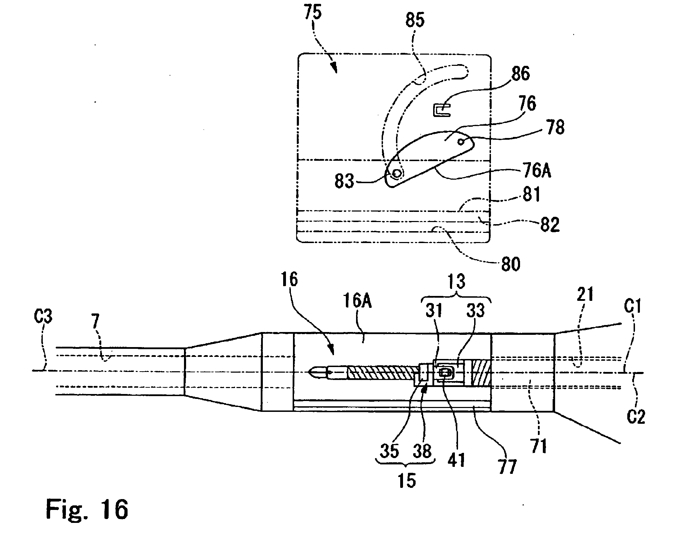

Then, as shown in FIG. 16, the operator removes the housing box 75.

With the endoscopic treatment instrument system 74, the first engagement mechanism 13 facilitates the engagement of the treatment instrument head 67 with the treatment instrument driving mechanism 71 by attaching the housing box 75 to the positioning member 77, and the second engagement mechanism 15 can connect the treatment instrument head 67 to the treatment instrument driving mechanism 71 reliably by the rotation of the treatment instrument head 67 using the swinging member 76. Also, the through hole 30 is easily aligned with the projection 32, solely with the insertion of the positioning member 77 into the first groove 80. Further by swinging the swinging member 76 in this condition, the treatment instrument head 67 can be swung reliably around the first engagement mechanism 13. Thereby, the second engagement mechanism 15 easily brings about the engagement, thereby permitting the treatment instrument head 67 to be easily attached to the treatment instrument driving mechanism 71.

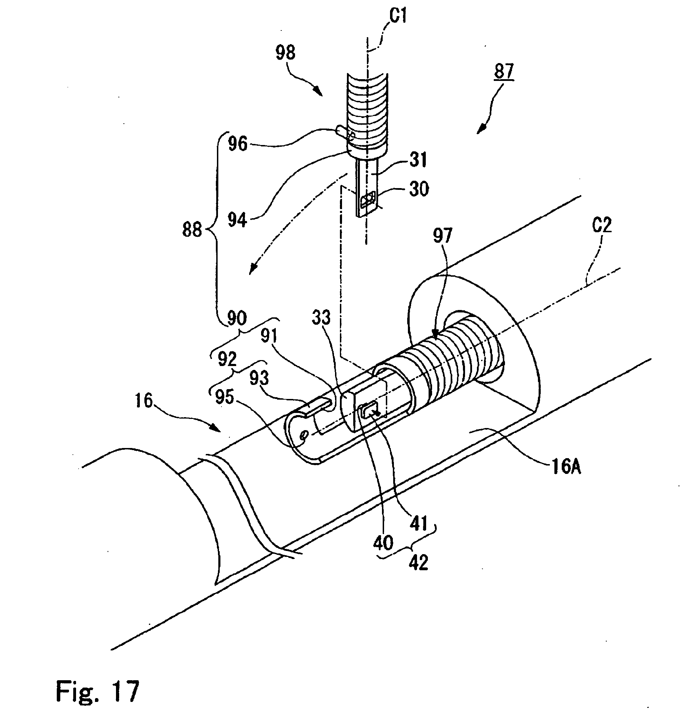

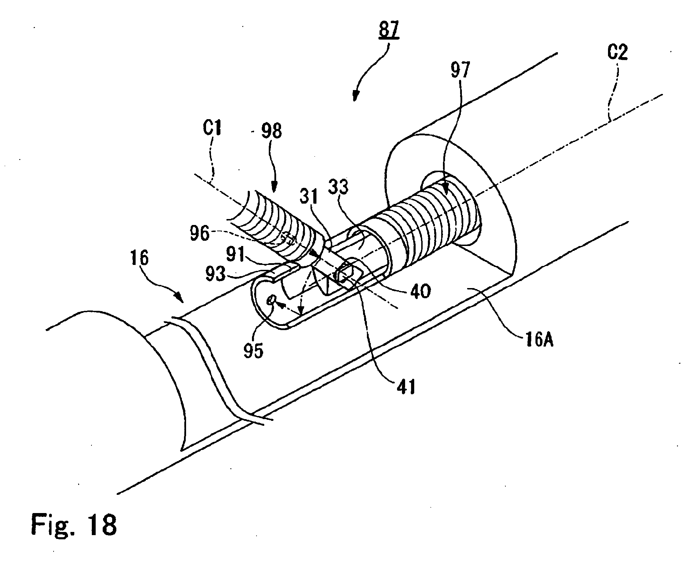

The following describes the fifth embodiment with reference to FIGS. 17 and 18.

Note that the same reference numerals have been used as in the foregoing other embodiments for similar parts and their description is therefore omitted.

The difference between the fifth embodiment and the first embodiment is that a notch portion 91 of the support 90 of the second engagement mechanism 88 in an endoscopic treatment instrument system 87 according to the fifth embodiment permits inserting an engagement portion 94 connected to a proximal end of the first sheath tube portion 3 from the protruding side of the projection 32 and the support 90 includes a restricting member 92 for restricting an axial movement of a flange 35.

The restricting member 92 has an inside diameter permitting the engagement portion 94 to be arranged in a covered condition and includes a ring member 93 having a length equal to or greater than the width of the engagement portion 94. The notch portion 91 is arranged in a protruding direction of a projection 32 of the ring member 93 and sized to permit an insertion of the engagement portion 94 into the ring member 93 from the radial direction.

An engagement hole 95 is arranged on the side face of the ring member 93 opposed to the notch portion 91 in such a way as to engage a pin 96 which protrudes radially along the axial direction of the through hole 30 from the outer peripheral surface of the engagement portion 94.

The through hole 30 is longer in a direction perpendicular to an axis of a treatment instrument head 98, and the head 42 of the projection 32 is longer in an axial direction of a treatment instrument driving mechanism 97.

The following describes an operating method of the endoscopic treatment instrument system 87 according to this embodiment.

Similarly to the first embodiment, an operator advances a distal end of the treatment instrument driving mechanism 97 from inside the through path 21 to an instrument insertion opening 16 and inserts the treatment instrument head 98 into the treatment instrument insertion opening 16 from the side of the first connecting member 31. At this point, a central axis C1 of the treatment instrument head 98 is substantially perpendicular to the central axis C2 of the treatment instrument driving mechanism 97. Thereby, the longer side of the through hole 30 coincides with that of a head 41 of the projection 32, enabling the through hole 30 of the first connecting member 31 to fit over the head 41 of the second connecting member 33. The operator then fits the head 41 into the through hole 30 and passes it through the through hole 30 to the position of the supporting shaft 40. As shown in FIG. 18, the operator then rotates the treatment instrument head 98 around the supporting shaft 40 until the central axis C1 of the treatment instrument head 98 becomes aligned with the central axis C2 of the treatment instrument driving mechanism 97, and until the engagement portion 94 abuts the ring member 93.

After the engagement portion 94 abuts the ring member 93, the operator further rotates the treatment instrument head 98 while bending it toward the side of the opening of the notch portion 91, inserts the engagement portion 94 into the ring member 93 from the notch portion 91 when the direction of a longer side of the through hole 30 becomes substantially perpendicular to that of the head 41, and inserts the pin 96 into an engagement hole 95 so as to engage the engagement portion 94 with the support 90.

At this point, both the first connecting member 31 and the second connecting member 33 are engaged with each other similarly to the above description, which attaches the treatment instrument head 98 to the treatment instrument driving mechanism 97.

With the endoscopic treatment instrument system 87, the same action and effects as in the first embodiment achieved. Moreover, the treatment instrument head 98 is attached or detached while it is bent toward the opening of the notch portion 91, thereby achieving a better connection between the treatment instrument head 98 and the treatment instrument driving mechanism 97.

The technical scope of the present invention is not limited to the foregoing preferred embodiments, and various modifications or changes can be made.

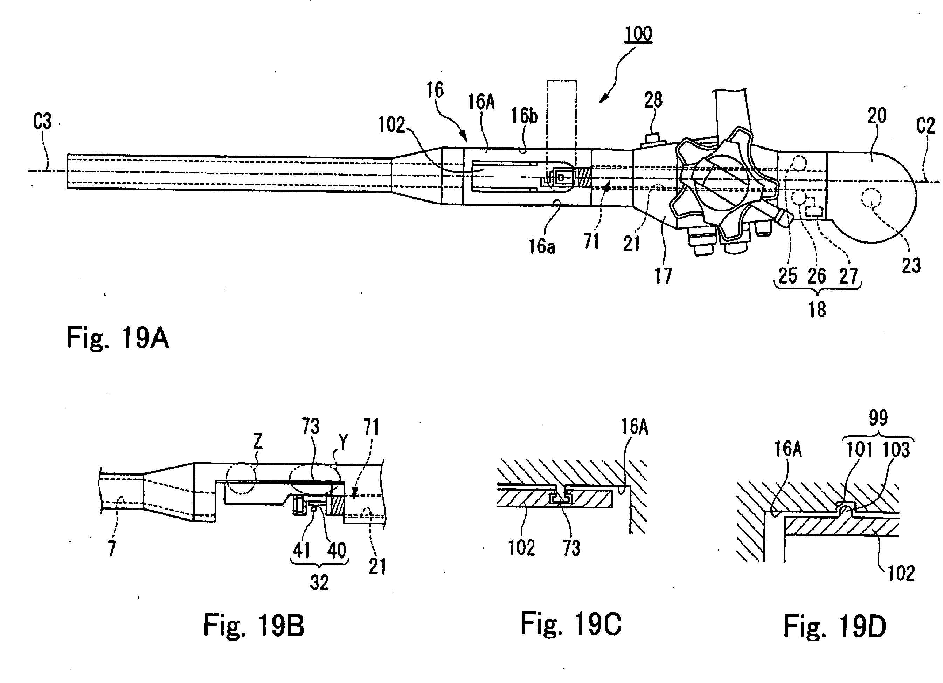

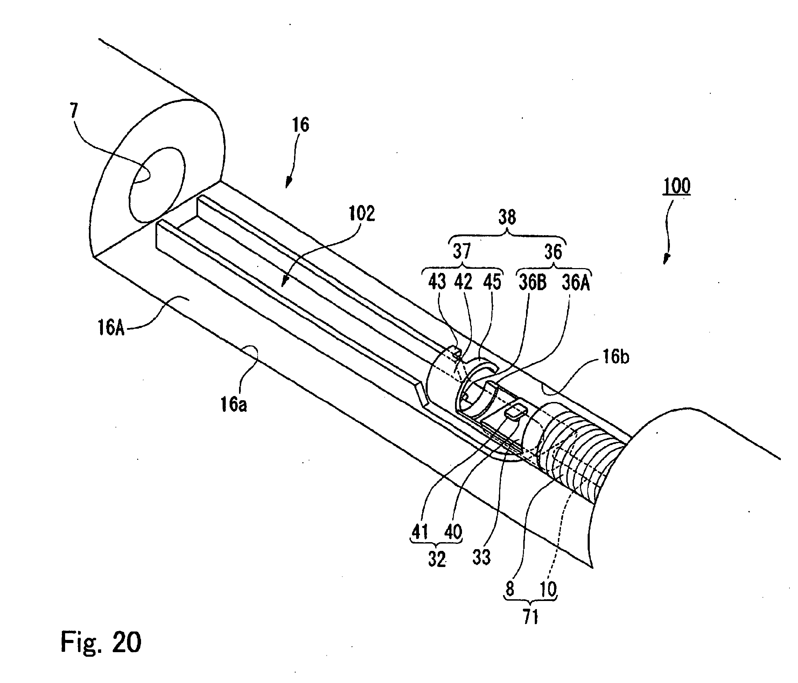

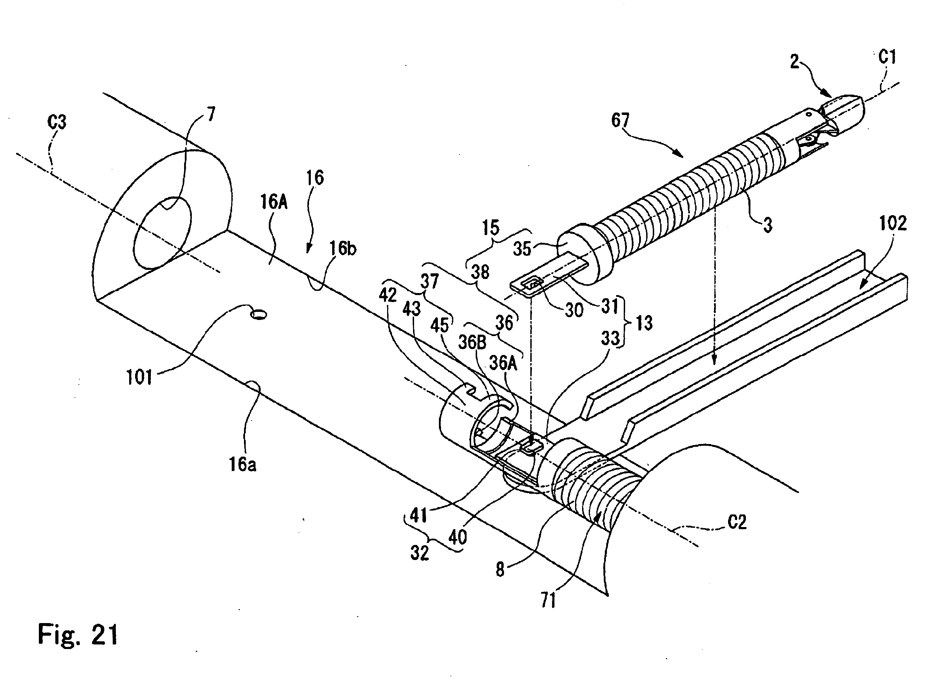

For example, in the third embodiment, the restricting member 72 is formed in plate shape and arranged in a standing condition with a height permitting abutment against the one-end side face 68a of the swinging member 68 on the side of the one end 16a of the wall portion 16A along the channel 7. As shown in FIGS. 19A to 21, however, an endoscopic treatment instrument system 100 can be provided with a restricting member formed of a click mechanism 99.

The click mechanism 99 includes a depression 101, which is arranged on a central axis C3 of a channel 7 and on the side of the channel 7 relative to the position where the turning shaft 73 is arranged, and a protrusion 103, which is arranged in a position where it can engage with the depression 101 when a central axis of a swinging member 102 coincides with a central axis C3 of the channel 7.

Where the protrusion 103 does not engage with the depression 101, the protrusion 103 can move on a wall portion 16A.

According to the endoscopic treatment instrument system 100, when a swinging member 102 is rotated up to the position of engaging the protrusion 103 with the depression 101, the click mechanism 99 restricts movement of the swinging member 102, thereby achieving the same action and effect as in the third embodiment.

While there has been shown and described what is considered to be preferred embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

## Claims

1. An endoscopic treatment instrument system (1, 46, 66, 74, 87, 100), comprising:
a treatment instrument head (6, 53, 67, 98) including:
a treatment portion (2) configured for conducting a treatment responsive to an external operation;
a first sheath tube portion (3, 48) configured for supporting the treatment portion at a distal end thereof; and
a first operating member (5) configured for driving the treatment portion through forward and backward movements relative to the first sheath tube portion (3, 48); and
an endoscope (12, 70) having a channel (7) configured for the passage therethrough of the treatment instrument head (6, 53, 67, 98);
the endoscopic treatment instrument system (1, 46, 66, 74, 87, 100) further comprising:
a treatment instrument driving mechanism (11, 52, 71, 97) including a second sheath tube portion (8, 50) moveable forward and backward within the channel (7) and engageable with the first sheath tube portion (3, 48);
a second operating member (10) moveable forward and backward within the second sheath tube portion (8, 50) and engageable with the first operating member (5), the treatment instrument driving mechanism being provided on said endoscope (12, 70);
a first engagement mechanism (13, 47) configured for coupling a proximal end of the first operating member (5) and a distal end of the second operating member (10), **characterized in that** the first engagement mechanism (13) includes:
a first connecting member (31, 60) connected to the proximal end of the first operating member (5) and having a through hole (30, 58); and
a second connecting member (33, 62) connected to the distal end of the second operating member (10) and having a projection (41, 61) which can fit into and pass through said through hole.

2. The endoscopic treatment instrument system according to claim 1, wherein the first engagement mechanism (13) is configured to enable coupling of the first and second operating members (5, 10) when a central axis of said treatment instrument driving mechanism (11, 52, 71, 97) intersects with that of said treatment instrument head (6, 53, 67, 98), and securing the coupling by engagement therebetween when their central axes become aligned with each other.

3. The endoscopic treatment instrument system according to claim 2, further comprising:
a second engagement mechanism (15, 88) configured for coupling the proximal end of the first sheath tube portion (3, 48) and the distal end of the second sheath tube portion (8, 50), the second engagement mechanism (15) enabling coupling when the central axis of the treatment instrument driving mechanism (11, 52, 71, 97) is aligned with that of the treatment instrument head (6, 53, 67, 98), with the first engagement mechanism (13) serving as a turning center.

4. The endoscopic treatment instrument system (74) according to claim 2, further comprising:
an instrument insertion opening (16) arranged at a proximal end of the channel (7) to enable said treatment instrument head (67) to be attached or detached to or from said treatment instrument driving mechanism (71);
an accommodating portion (75) configured for accommodating said treatment instrument head (67);
a swinging member (76) rotatably arranged in the accommodating portion (75), holding said treatment instrument head (67), and swinging with the treatment instrument head (67); and
a positioning member (77) configured for positioning the accommodating portion (75) at the instrument insertion opening (16) so that said treatment instrument head (67) swings with the swinging member (76) around the first engagement mechanism (13).

5. The endoscopic treatment instrument system according to claim 2, further comprising:
a swinging member (68, 76, 102) arranged in the endoscope (12, 70) and rotatable while holding said treatment instrument head (53, 67, 98) around the first engagement mechanism (13).

6. The endoscopic treatment instrument system according to claim 5, further comprising:
a restricting member (72, 99) for restricting swinging of the swinging member (68, 75) beyond a position where the central axis of said treatment instrument head (67, 98) is aligned with the central axis of said treatment instrument driving mechanism (71, 97).

7. The endoscopic treatment instrument system (1) according to claim 3, wherein:
the through hole (30) of the first engagement mechanism (13) is formed longer in the axial direction of said treatment instrument head (6), and
the projection of the first engagement mechanism (13) is formed longer in the direction perpendicular to the axial direction of said treatment instrument driving mechanism (11) and able to pass through the through hole (30); and said second engagement mechanism (15) includes:
an engagement portion (35) provided at the proximal end of the first sheath tube portion (3),
a notch portion (36) provided at the distal end of the second sheath tube portion (8) and capable of being inserted into the engagement portion (35) from a turning direction of said treatment instrument head (6), and
a restricting member (37) engageable with the engagement portion (35) and restricting axial movement of the engagement portion (35).

8. The endoscopic treatment instrument system (66) according to claim 3, wherein:
the through hole (30) is formed longer in the direction perpendicular to the axial direction of said treatment instrument head (67), and
the projection (41) is formed longer in the axial direction of said treatment instrument driving mechanism (52) and able to pass through the through hole (30); and
said second engagement mechanism (15) includes:
an engagement portion (35) provided at the proximal end of the first sheath tube portion (3),
a notch portion (36) provided at the distal end of the second sheath tube portion (8) and capable of being inserted into the engagement portion (35) from a turning direction of said treatment instrument head (67), and
a restricting member (37, 72) engageable with the engagement portion (35) and restricting an axial movement of the engagement portion (35).

9. The endoscopic treatment instrument system (74) according to claim 3, wherein:
the through hole (30) is formed longer in the axial direction of said treatment instrument head (67), and
the projection (31) is formed longer in the direction perpendicular to the axial direction of said treatment instrument driving mechanism (71) and protruding in such a way as to enable engagement with the through hole (30); and
said second engagement mechanism (15) includes:
an engagement portion (35) provided at the proximal end of the first sheath tube portion (3),
a notch portion (36A, 36B) provided at the distal end of the second sheath tube portion (8) and capable of being inserted into the engagement portion (35) from a protruding direction of the projection (31), and
a restricting member (37, 72) restricting axial movement of the engagement portion.

10. The endoscopic treatment instrument system (87) according to claim 3, wherein:
the through hole (30) is formed longer in a direction perpendicular to the axial direction of said treatment instrument head (98), and
the projection (31) is formed longer in the axial direction of said treatment instrument driving mechanism (97) and protruding in such a way as to enable its engagement with the through hole (30); and
said second engagement mechanism (88) includes:
an engagement portion (94) provided at the proximal end of the first sheath tube portion,
a notch portion (91) provided at the distal end of the second sheath tube portion (8) and capable of being inserted into the engagement portion from a protruding direction of the projection (31), and
a restricting member (92) restricting axial movement of the engagement portion (94).

11. The endoscopic treatment instrument system according to a combination of claims 1 to 3, the endoscopic system further comprising a side opening (16) provided at a proximal region of the endoscope insertion portion,
wherein:
the channel (7) of the endoscope (12, 70) extends axially; and
the treatment instrument driving mechanism (11, 52, 71, 97) is capable of positioning the treatment instrument head (6, 53, 67, 98) such that the first and second engagement mechanisms (13, 15, 88) are accessible at the side opening (16), to enable replacement of the instrument head (6, 53, 67, 98) with another instrument head (6, 53, 67, 98) without removing the treatment instrument from the channel (7) of the endoscope (12, 70).

12. The endoscopic system of claim 11, wherein the treatment instrument driving mechanism (11, 52, 71, 97) configured for moving the treatment instrument in the channel (7) is electrically operable.

13. The endoscopic system of claim 12, wherein the first and second engagement mechanisms (13, 15, 88) are configured to enable interconnection of the first and second sheath tube portions (3, 8, 48, 50) and to interconnect the first and second operating members (5, 10) when a distal region of the treatment instrument is positioned at the side opening of the endoscope (12, 70).

14. The endoscopic system of claim 13, wherein the first and second engagement mechanisms (13, 15, 88) are structured such that the treatment instrument head is attachable to the treatment instrument when oriented such that their axial directions are substantially perpendicular to one another and such that they become interlocked when the treatment instrument head is pivoted to align their axial directions.

15. The endoscopic system of claim 13, wherein the first and second operating members (5, 10) comprise respective operating wires (5, 10), respectively, and wherein reciprocal movement of the operating wires (5, 10) causes the treatment instrument head (6, 53, 67, 98) to alternate between first and second operational modes.

16. The endoscopic system of claim 11, wherein the first and second engagement mechanisms (13, 15, 88) include a swingable carrier (68, 75) on which the treatment instrument head is placeable and with which it is swingable into an interconnected and aligned position within the channel (7) of the endoscope (12, 70).

17. The endoscopic system of claim 16, wherein the swingable carrier (68, 75) comprises a detent which is registrable with an opening in the endoscope channel (7) to align the swingable carrier (68, 75) and the treatment instrument head with the axial direction of the channel (7).

18. The endoscopic system of claim 11, including a housing (75) which is capable of holding the treatment instrument head and of being placed over the side opening (16) of the channel (7) and which includes a lever that enables swinging of the treatment instrument head into a locked position at the side opening (16) of the endoscope channel (7).

19. The endoscopic system of claim 13, wherein the first and second engagement mechanisms (13, 15, 88) are configured for connecting the first and second sheath tube portions (3, 8, 48, 50) and comprise a ring arrangement (43, 45) which snappingly receives and holds a round flange (35) provided on one of the first and second sheath tube portions (3, 8, 48, 50).

20. The endoscopic system of claim 12, wherein the electrically operable drive mechanism (18) comprises a pair of rollers (25, 26).

21. The endoscopic system of claim 12, including a holding chamber (20) in which the treatment instrument is capable of being stored while being withdrawn from the channel (7) of the endoscope (12, 70).

22. The endoscopic system of claim 12, including at least one switch control (27) for operating the operating members (5, 10) and the drive mechanism for moving the treatment instrument head slidingly back and forth within the channel (7).

## Patentansprüche

1. Endoskopisches Behandlungsinstrumentensystem (1, 46, 66, 74, 87, 100) umfassend:
einen Behandlungsinstrumentenkopf (6, 53, 67, 98) mit:
einem Behandlungsabschnitt (2), der dazu eingerichtet ist, in Reaktion auf eine externe Bedienung eine Behandlung durchzuführen;
einem ersten Hülsenrohrabschnitt (3, 48), der dazu eingerichtet ist, den Behandlungsabschnitt an dessen distalem Ende zu unterstützen; und
einem ersten Bedienelement (5), das dazu eingerichtet ist, den Behandlungsabschnitt durch Vorwärts- und Rückwärtsbewegungen relativ zu dem ersten Hülsenrohrabschnitt (3, 48) anzutreiben; und
Endoskop (12, 70) mit einem Kanal (7), der dazu eingerichtet ist, die Durchführung des Behandlungsinstrumentenkopfs (6, 53, 67, 98) zu ermöglichen;
wobei das endoskopische Behandlungsinstrumentensystem (1, 46, 66, 74, 87, 100) ferner umfasst:
einen Behandlungsinstrumentenantriebsmechanismus (11, 52, 71, 97), der einen zweiten Hülsenrohrabschnitt (8, 50) umfasst, der vorwärts und rückwärts innerhalb des Kanals (7) bewegbar und mit dem ersten Hülsenrohrabschnitt (3, 48) in Eingriff bringbar ist;
ein zweites Bedienelement (10), das vorwärts und rückwärts innerhalb des zweiten Hülsenrohrabschnitts (8, 50) bewegbar und mit dem ersten Bedienelement (5) in Eingriff bringbar ist, wobei der Behandlungsinstrumentenantriebsmechanismus an dem Endoskop (12, 70) vorgesehen ist;
einen ersten Kopplungsmechanismus (13, 47), der dazu eingerichtet ist, ein proximales Ende des ersten Bedienelements (5) und ein distales Ende des zweiten Bedienelements (10) zu koppeln,
**dadurch gekennzeichnet, dass** der erste Kopplungsmechanismus (13) umfasst:
ein erstes Verbindungselement (31, 60), das mit dem proximalen Ende des ersten Bedienelements (5) verbunden ist und einen Durchlass (30, 58) aufweist;
ein zweites Verbindungselement (33, 62) das mit dem distalen Ende des zweiten Bedienelemente (10) verbunden ist und einen Vorsprung (41, 61) aufweist, der in den Durchlass hinein und hindurch passt.

2. Endoskopisches Behandlungsinstrumentensystem gemäß Anspruch 1, wobei der erste Kopplungsmechanismus (13) dazu eingerichtet ist, eine Kopplung zwischen dem ersten und dem zweiten Bedienelement (5, 10) zu ermöglichen, wenn eine zentrale Achse des Behandlungsinstrumentenantriebsmechanismus (11, 52, 71, 97) diejenige des Behandlungsinstrumentenkopfs (6, 53, 67, 98) schneidet, und die Kopplung durch eine Verbindung zwischen den beiden zu sichern, wenn ihre zentralen Achsen miteinander fluchten.

3. Endoskopisches Behandlungsinstrumentensystem gemäß Anspruch 2, das ferner umfasst:
einen zweiten Kopplungsmechanismus (15, 88), der dazu eingerichtet ist, das proximale Ende des ersten Hülsenrohrabschnitts (3, 48) und das distale Ende des zweiten Hülsenrohrabschnitts (8, 50) zu koppeln, wobei der zweite Kopplungsmechanismus (15) die Kopplung ermöglicht, wenn die zentrale Achse des Behandlungsinstrumentenantriebsmechanismus (11, 52, 71, 97) mit derjenigen des Behandlungsinstrumentenkopfs (6, 53, 67, 98) fluchtet, wobei der erste Kopplungsmechanismus (13) als Drehzentrum dient.

4. Endoskopisches Behandlungsinstrumentensystem (74) gemäß Anspruch 2, das ferner umfasst:
eine Instrumenteneinführöffnung (16), die an einem proximalen Ende des Kanals (7) angeordnet ist, um zu ermöglichen, den Behandlungsinstrumentenkopf (67) an dem Behandlungsinstrumentenantriebsmechanismus (71) zu befestigen oder davon zu lösen;
einen Aufnahmeabschnitt (75), der zur Aufnahme des
Behandlungsinstrumentenkopfs (67) eingerichtet ist;
ein Schwingungselement (76), das drehbar in dem Aufnahmeabschnitt (75) angeordnet ist, den Behandlungsinstrumentenkopf (67) hält und mit dem Behandlungsinstrumentenkopf (67) schwingt;
ein Positionierungselement (77), das dazu eingerichtet ist, den Aufnahmeabschnitt (75) an der Instrumenteneinführöffnung (16) zu positionieren, so dass der Behandlungsinstrumentenkopf (67) mit dem Schwingungselement (76) um den ersten Kopplungsmechanismus (13) schwingt.

5. Endoskopisches Behandlungsinstrumentensystem gemäß Anspruch 2, das ferner umfasst:
ein Schwingungselement (68, 76, 102), das in dem Endoskop (12, 70) angeordnet und um den ersten Kopplungsmechanismus (13) drehbar ist, während es den Behandlungsinstrumentenkopf (53, 67, 98) hält.

6. Endoskopisches Behandlungsinstrumentensystem gemäß Anspruch 5, das ferner umfasst:
ein Beschränkungselement (72, 99) zur Beschränkung der Schwingung des Schwingungselements (68, 75) über eine Position hinaus, wo die zentrale Achse des Behandlungsinstrumentenkopfs (67, 98) mit der zentralen Achse des Behandlungsinstrumentenantriebsmechanismus (71, 97) fluchtet.

7. Endoskopisches Behandlungsinstrumentensystem (1) gemäß Anspruch 3, wobei:
der Durchlass (30) des ersten Kopplungsmechanismus (13) in der axialen Richtung des Behandlungsinstrumentenkopfs (6) länger geformt ist, und
der Vorsprung des ersten Kopplungsmechanismus (13) in der Richtung senkrecht zu der axialen Richtung des Behandlungsinstrumentenantriebsmechanismus (11) länger geformt und fähig ist, durch den Durchlass (30) hindurchgeführt zu werden; und
der zweite Kopplungsmechanismus (15) umfasst:
einen Kopplungsabschnitt (35), der an dem proximalen Ende des ersten Hülsenrohrabschnitts (3) vorgesehen ist,
einen Nutabschnitt (36), der an dem distalen Ende des zweiten Hülsenrohrabschnitts (8) vorgesehen und so ausgeführt ist, dass er aus einer Drehrichtung des Behandlungsinstrumentenkopfs (6) in den Kopplungsabschnitt (35) eingeführt werden kann, und
ein Beschränkungselement (37), das mit dem Kopplungsabschnitt (35) verbindbar ist und eine axiale Bewegung des Kopplungsabschnitts (35) beschränkt.

8. Endoskopisches Behandlungsinstrumentensystem (66) gemäß Anspruch 3, wobei:
der Durchlass (30) in der Richtung senkrecht zu der axialen Richtung des Behandlungsinstrumentenkopfs (67) länger geformt ist, und
der Vorsprung (41) in der axialen Richtung des
Behandlungsinstrumentenantriebsmechanismus (52) länger geformt und fähig ist, durch den Durchlass (30) hindurchgeführt zu werden; und
der zweite Kopplungsmechanismus (15) umfasst:
einen Kopplungsabschnitt (35), der an dem proximalen Ende des ersten Hülsenrohrabschnitts (3) vorgesehen ist,
einen Nutabschnitt (36), der an dem distalen Ende des zweiten Hülsenrohrabschnitts (8) vorgesehen und so ausgeführt ist, dass er aus einer Drehrichtung des Behandlungsinstrumentenkopfs (67) in den Kopplungsabschnitt (35) eingeführt werden kann, und
ein Beschränkungselement (37, 72), das mit dem Kopplungsabschnitt (35) verbindbar ist und eine axiale Bewegung des Kopplungsabschnitts (35) beschränkt.

9. Endoskopisches Behandlungsinstrumentensystem (74) gemäß Anspruch 3, wobei:
der Durchlass (30) in der axialen Richtung des Behandlungsinstrumentenkopfs (67) länger geformt ist, und
der Vorsprung (31) in der Richtung senkrecht zu der axialen Richtung des Behandlungsinstrumentenantriebsmechanismus (71) länger geformt ist und derart vorsteht, dass eine Kopplung mit dem Durchlass (30) möglich ist; und
der zweite Kopplungsmechanismus (15) umfasst:
einen Kopplungsabschnitt (35), der an dem proximalen Ende des ersten Hülsenrohrabschnitts (3) vorgesehen ist,
einen Nutabschnitt (36A, 36B), der an dem distalen Ende des zweiten Hülsenrohrabschnitts (8) vorgesehen und so ausgeführt ist, dass er aus einer Vorstehrichtung des Vorsprungs (31) in den Kopplungsabschnitt (35) eingeführt werden kann, und
ein Beschränkungselement (37, 72), das eine axiale Bewegung des Kopplungsabschnitts beschränkt.

10. Endoskopisches Behandlungsinstrumentensystem (87) gemäß Anspruch 3, wobei:
der Durchlass (30) in der Richtung senkrecht zu der axialen Richtung des Behandlungsinstrumentenkopfs (98) länger geformt ist, und
der Vorsprung (31) in der axialen Richtung des
Behandlungsinstrumentenantriebsmechanismus (97) länger geformt ist und derart vorsteht, dass seine Kopplung mit dem Durchlass (30) möglich ist; und
der zweite Kopplungsmechanismus (88) umfasst:
einen Kopplungsabschnitt (94), der an dem proximalen Ende des ersten Hülsenrohrabschnitts vorgesehen ist,
einen Nutabschnitt (91), der an dem distalen Ende des zweiten Hülsenrohrabschnitts (8) vorgesehen und so ausgeführt ist, dass er aus einer Vorstehrichtung des Vorsprungs (31) in den Kopplungsabschnitt eingeführt werden kann, und
ein Beschränkungselement (92), das eine axiale Bewegung des Kopplungsabschnitts (94) beschränkt.

11. Endoskopisches Behandlungsinstrumentensystem gemäß einer Kombination der Ansprüche 1 bis 3, wobei das endoskopische System ferner eine Seitenöffnung (16) umfasst, die an einem proximalen Bereich, des Endoskopeinführabschnitts vorgesehen ist, wobei:
sich der Kanal (7) des Endoskops (12, 70) axial erstreckt; und
der Behandlungsinstrumentenantriebsmechanismus (11, 52, 71, 97) dazu in der Lage ist, den Behandlungsinstrumentenkopf (6, 53, 67, 98) derart zu positionieren, dass der erste und der zweite Kopplungsmechanismus (13, 15, 88) an der Seitenöffnung (16) zugänglich sind, um einen Austausch des Instrumentenkopfes (6, 53, 67, 98) durch einen anderen Instrumentenkopf (6, 53, 67, 98) zu ermöglichen, ohne das Behandlungsinstrument aus dem Kanal des Endoskops (12, 72) zu entfernen.

12. Endoskopisches System gemäß Anspruch 11, wobei der Behandlungsinstrumentenantriebsmechanismus (11, 52, 71, 97), der dazu eingerichtet ist, das Behandlungsinstrument in dem Kanal (7) zu bewegen, elektrisch betätigbar ist.

13. Endoskopisches System gemäß Anspruch 12, wobei der erste und der zweite Kopplungsmechanismus (13, 15, 88) dazu eingerichtet sind, eine Verbindung zwischen dem ersten und dem zweiten Hülsenrohrabschnitt (3, 8, 48, 50) zu ermöglichen und das erste und das zweite Bedienelement (5, 10) zu verbinden, wenn ein distaler Bereich des Behandlungsinstruments an der Seitenöffnung des Endoskops (12, 70) positioniert ist.

14. Endoskopisches System gemäß Anspruch 13, wobei der erste und der zweite Kopplungsmechanismus (13, 15, 88) derart aufgebaut sind, dass der Behandlungsinstrumentenkopf an dem Behandlungsinstrument befestigbar ist, wenn sie derart orientiert sind, dass ihre axialen Richtungen im Wesentlichen senkrecht zueinander sind und dass sie miteinander verriegelt werden, wenn der Behandlungsinstrumentenkopf geschwenkt wird, um ihre axialen Richtungen auszurichten.

15. Endoskopisches System gemäß Anspruch 13, wobei das erste und das zweite Bedienelement (5, 10) jeweilige Betätigungsdrähte (5, 10) umfassen, und wobei eine reziproke Bewegung der Betätigungsdrähte (5, 10) den Behandlungsinstrumentenkopf (6, 53, 67, 98) dazu veranlasst, zwischen einem ersten und einem zweiten Betriebsmodus zu wechseln.

16. Endoskopisches System gemäß Anspruch 11, wobei der erste und der zweite Kopplungsmechanismus (13, 15, 88) einen schwingfähigen Träger (68, 75) umfassen, auf dem der Behandlungsinstrumentenkopf platzierbar ist und mit dem er in eine verbundene und ausgerichtete Position innerhalb des Kanals (7) des Endoskops (12, 70) schwingfähig ist.

17. Endoskopisches System gemäß Anspruch 16, wobei der schwingfähige Träger (68, 75) ein Rastelement umfasst, welches mit einer Öffnung in dem Endoskopkanal (7) verrastbar ist, um den schwingfähigen Träger (68, 75) und den Behandlungsinstrumentenkopf mit der axialen Richtung des Kanals (7) auszurichten.

18. Endoskopisches System gemäß Anspruch 11, mit einem Gehäuse (75), das dazu in der Lage ist, den Behandlungsinstrumentenkopf zu halten und über der Seitenöffnung (16) des Kanals (7) platziert zu werden und das einen Hebel umfasst, der es dem Behandlungsinstrumentenkopf ermöglicht, in eine verriegelte Position an der Seitenöffnung (16) des Endoskopskanals (7) zu schwingen.

19. Endoskopisches System gemäß Anspruch 13, wobei der erste und der zweite Kopplungsmechanismus (13, 15, 88) dazu eingerichtet sind, den ersten und den zweiten Hülsenrohrabschnitt (3, 8, 48, 50) zu verbinden und eine Ringanordnung (43, 45) umfassen, die einen runden Flansch (35) einrastend aufnimmt und hält, der an dem ersten oder dem zweiten Hülsenrohrabschnitt (3, 8, 48, 50) vorgesehen ist.

20. Endoskopisches System gemäß Anspruch 20, wobei der elektrisch betätigbare Antriebsmechanismus (18) ein Paar von Rollen (25, 26) umfasst.

21. Endoskopisches System gemäß Anspruch 12, mit einer Aufnahmekammer (20), in der das Behandlungsinstrument aufgenommen werden kann, während es von dem Kanal (7) des Endoskops (12, 70) zurückgezogen wird.

22. Endoskopisches System gemäß Anspruch 12, mit mindestens einer Schaltersteuerung (27) zum Betätigen der Bedienelemente (5, 10) und des Antriebsmechanismus, um den Behandlungsinstrumentenkopf innerhalb des Kanals (7) rückwärts und vorwärts zu schieben.

## Revendications

1. Système (1, 46, 66, 74, 87, 100) d'instrument de traitement endoscopique, comprenant :
une tête (6, 53, 67, 98) d'instrument de traitement incluant :
une partie (2) de traitement configurée pour réaliser un traitement en réponse à une opération externe ;
une première partie (3, 48) de tube de gainage configurée pour supporter la partie de traitement au niveau d'une extrémité distale de celle-ci ;
et
un premier élément (5) d'actionnement configuré pour entraîner la partie de traitement par l'intermédiaire de mouvements de va-et-vient par rapport à la première partie (3, 48) de tube de gainage ; et
un endoscope (12, 70) comportant un canal (7) configuré pour le passage à travers celui-ci de la tête (6, 53, 67, 98) d'instrument de traitement ;
le système (1, 46, 66, 74, 87, 100) d'instrument de traitement endoscopique comprenant en outre :
un mécanisme (11, 52, 71, 97) d'entraînement d'instrument de traitement incluant une deuxième partie (8, 50) de tube de gainage mobile vers l'avant et vers l'arrière à l'intérieur du canal (7) et engageable avec la première partie (3, 48) de tube de gainage ;
un deuxième élément (10) d'actionnement mobile vers l'avant et vers l'arrière à l'intérieur de la deuxième partie (8, 50) de tube de gainage et engageable avec le premier élément (5) d'actionnement, le mécanisme d'entraînement d'instrument de traitement étant prévu sur ledit endoscope (12, 70) ;
un premier mécanisme (13, 47) d'engagement configuré pour coupler une extrémité proximale du premier élément (5) d'actionnement et une extrémité distale du deuxième élément (10) d'actionnement, **caractérisé en ce que** le premier mécanisme (13) d'actionnement comprend :
un premier élément (31, 60) de connexion connecté à l'extrémité proximale du premier élément (5) d'actionnement et comportant un trou traversant (30, 58) ; et
un deuxième élément (33, 62) de connexion connecté à l'extrémité distale du deuxième élément (10) d'actionnement et comportant une saillie (41, 61) qui peut s'ajuster dans et passer à travers ledit trou traversant.

2. Système d'instrument de traitement endoscopique selon la revendication 1, dans lequel le premier mécanisme (13) d'engagement est configuré pour permettre le couplage des premier et deuxième éléments (5, 10) d'actionnement lorsqu'un axe central dudit mécanisme (11, 52, 71, 97) d'entraînement d'instrument de traitement croise celui de ladite tête (6, 53, 67, 98) d'instrument de traitement, et la sécurisation du couplage par engagement entre eux lorsque leurs axes deviennent alignés l'un par rapport à l'autre.

3. Système d'instrument de traitement endoscopique selon la revendication 2, comprenant en outre :
un deuxième mécanisme (15, 88) d'engagement configuré pour coupler l'extrémité proximale de la première partie (3, 48) de tube de gainage et l'extrémité distale de la deuxième partie (8, 50) de tube de gainage, le deuxième mécanisme (15) d'engagement permettant le couplage lorsque l'axe central du mécanisme (11, 52, 71, 97) d'entraînement d'instrument de traitement est aligné avec celui de la tête (6, 53, 67, 98) d'instrument de traitement, le premier mécanisme (13) d'engagement servant de centre de rotation.

4. Système (74) d'instrument de traitement endoscopique selon la revendication 2, comprenant en outre :
une ouverture (16) d'insertion d'instrument agencée au niveau d'une extrémité proximale du canal (7) pour permettre que ladite tête (67) d'instrument de traitement soit attachée ou détachée audit ou dudit mécanisme (71) d'entraînement d'instrument de traitement ;
une partie (75) de réception configurée pour recevoir ladite tête (67) d'instrument de traitement ;
un élément (76) oscillant disposé en rotation dans la partie (75) de réception, maintenant ladite tête (67) d'instrument de traitement, et oscillant avec la tête (67) d'instrument de traitement ; et
un élément (77) de positionnement configuré pour positionner la partie (75) de réception au niveau de l'ouverture (16) d'insertion d'instrument de sorte que ladite tête (67) d'instrument de traitement oscille avec l'élément (76) oscillant autour du premier mécanisme (13) d'engagement.

5. Système d'instrument de traitement endoscopique selon la revendication 2, comprenant en outre :
un élément (68, 76, 102) oscillant disposé dans l'endoscope (12, 70) et rotatif tout en maintenant ladite tête (53, 67, 98) d'instrument de traitement autour du premier mécanisme (13) d'engagement.

6. Systèmes d'instrument de traitement endoscopique selon la revendication 5, comprenant en outre :
un élément (72, 99) de limitation destiné à limiter l'oscillation de l'élément (68, 75) oscillant au-delà d'une position dans laquelle l'axe central de ladite tête (67, 98) d'instrument de traitement est aligné avec l'axe central dudit mécanisme (71, 97) d'entraînement d'instrument de traitement.

7. Système (1) d'instrument de traitement endoscopique selon la revendication 3, dans lequel :
le trou traversant (30) du premier mécanisme (13) d'engagement est formé plus long dans la direction axiale de ladite tête (6) d'instrument de traitement, et
la saillie du premier mécanisme (13) d'engagement est formée plus longue dans la direction perpendiculaire à la direction axiale dudit mécanisme (11) d'entraînement d'instrument de traitement et capable de passer à travers le trou traversant (30) ; et
ledit deuxième mécanisme (15) d'engagement comprend :
une partie d'engagement (35) prévue au niveau de l'extrémité proximale de la première partie (3) de tube de gainage,
une partie (36) d'encoche prévue au niveau de l'extrémité distale de la deuxième partie (8) de tube de gainage et capable d'être insérée dans la partie (35) d'engagement à partir d'un sens de rotation de ladite tête (6) d'instrument de traitement, et
un élément (37) de limitation engageable avec la partie (35) d'engagement et limitant le mouvement axial de la partie (35) d'engagement.

8. Système (66) d'instrument de traitement endoscopique selon la revendication 3, dans lequel :
le trou traversant (30) est formé plus long dans la direction perpendiculaire à la direction axiale de ladite tête (67) d'instrument de traitement, et
la saillie (41) est formée plus longue dans la direction axiale dudit mécanisme (52) d'entraînement d'instrument de traitement et capable de passer à travers le trou traversant (30) ; et
ledit deuxième mécanisme (15) d'engagement comprend :
une partie (35) d'engagement prévue au niveau de l'extrémité proximale de la première partie (3) de tube de gainage,
une partie (36) d'encoche prévue au niveau de l'extrémité distale de la deuxième partie (8) de tube de gainage et capable d'être insérée dans la partie (35) d'engagement à partir d'un sens de rotation de ladite tête (67) d'instrument de traitement, et
un élément (37, 72) de limitation engageable avec la partie (35) d'engagement et limitant un mouvement axial de la partie (35) d'engagement.

9. Système (74) d'instrument de traitement endoscopique selon la revendication 3, dans lequel :
le trou traversant (30) est formé plus long dans la direction axiale de ladite tête (67) d'instrument de traitement, et
la saillie (31) est formée plus longue dans la direction perpendiculaire à la direction axiale dudit mécanisme (71) d'entraînement d'instrument de traitement et faisant saillie de façon à permettre l'engagement avec le trou traversant (30) ; et
ledit deuxième mécanisme (15) d'engagement comprend :
une partie (35) d'engagement prévue au niveau de l'extrémité proximale de la première partie (3) de tube de gainage,
une partie (36A, 36B) d'encoche prévue au niveau de l'extrémité distale de la deuxième partie (8) de tube de gainage et capable d'être insérée dans la partie (35) d'engagement à partir d'une direction de saillie de la saillie (31), et
un élément (37, 72) de limitation limitant le mouvement axial de la partie d'engagement.

10. Système (87) d'instrument de traitement endoscopique selon la revendication 3, dans lequel :
le trou traversant (30) est formé plus long dans une direction perpendiculaire à la direction axiale de ladite tête (98) d'instrument de traitement, et
la saillie (31) est formée plus longue dans la direction axiale dudit mécanisme (97) d'entraînement d'instrument de traitement et faisant saillie de façon à permettre son engagement avec le trou traversant (30) ; et
ledit deuxième mécanisme (88) d'engagement comprend :
une partie (94) d'engagement prévue au niveau de l'extrémité proximale de la première partie de tube de gainage,
une partie (91) d'encoche prévue au niveau de l'extrémité distale de la deuxième partie (8) de tube de gainage et capable d'être insérée dans la partie d'engagement à partir d'une direction de saillie de la saillie (31), et
un élément (92) de limitation limitant le mouvement axial de la partie (94) d'engagement.

11. Système d'instrument de traitement endoscopique selon une combinaison des revendications 1 à 3, le système endoscopique comprenant en outre une ouverture latérale (16) prévue au niveau d'une région proximale de la partie d'insertion d'endoscope, dans lequel
le canal (7) de l'endoscope (12, 70) s'étend de manière axiale ; et
le mécanisme (11, 52, 71, 97) d'entraînement d'instrument de traitement est capable de positionner la tête (6, 53, 67, 98) d'instrument de traitement d'une manière telle que les premier et deuxième mécanismes (13, 15, 88) d'engagement sont accessibles au nouveau de l'ouverture latérale (16), pour permettre le remplacement de la tête (6, 53, 67, 98) d'instrument par une autre tête (6, 53, 67, 98) d'instrument sans enlever l'instrument de traitement du canal (7) de l'endoscope (12, 70).

12. Système endoscopique selon la revendication 11, dans lequel le mécanisme (11, 52, 71, 97) d'entraînement d'instrument de traitement configuré pour déplacer l'instrument de traitement dans le canal (7) peut être actionné électriquement.

13. Système endoscopique selon la revendication 12, dans lequel les premier et deuxième mécanismes (13, 15, 88) d'engagement sont configurés pour permettre une interconnexion des première et deuxième parties (3, 8, 48, 50) de tubes de gainage et pour interconnecter les premier et deuxième éléments (5, 10) d'actionnement lorsqu'une région distale de l'instrument de traitement est positionnée au niveau de l'ouverture latérale de l'endoscope (12, 70).

14. Système endoscopique selon la revendication 13, dans lequel les premier et deuxième mécanismes (13, 15, 88) d'engagement sont structurés d'une manière telle que la tête d'instrument de traitement est attachable à l'instrument de traitement lorsqu'ils sont orientés d'une manière telle que leurs directions axiales sont sensiblement perpendiculaires l'une par rapport à l'autre et d'une manière telle qu'ils deviennent interverrouillés lorsque la tête d'instrument de traitement est pivotée pour aligner leurs directions axiales.

15. Système endoscopique selon la revendication 13, dans lequel les premier et deuxième éléments (5, 10) d'actionnement comprennent des câbles d'actionnement respectifs (5, 10), respectivement, et dans lequel un mouvement de va-et-vient des câbles d'actionnement (5, 10) amène la tête (6, 53, 67, 98) d'instrument de traitement à alterner entre des premier et deuxième modes opérationnels.

16. Système endoscopique selon la revendication 11, dans lequel les premier et deuxième mécanismes (13, 15, 88) d'engagement comprennent un support oscillant (68, 75) sur lequel la tête d'instrument de traitement peut être placée et avec lequel elle peut osciller dans une position interconnectée et alignée à l'intérieur du canal (7) de l'endoscope (12, 70).

17. Système endoscopique selon la revendication 16, dans lequel le support oscillant (68, 75) comprend un cran qui est enregistrable avec une ouverture dans le canal (7) de l'endoscope pour aligner le support oscillant (68, 75) et la tête d'instrument de traitement avec la direction axiale du canal (7).

18. Système endoscopique selon la revendication 11, comprenant un logement (75) qui est capable de maintenir la tête d'instrument de traitement et d'être placé sur l'ouverture latérale (16) du canal (7) et qui comprend un levier qui permet l'oscillation de la tête d'instrument de traitement dans une position verrouillée au niveau de l'ouverture latérale (16) du canal (7) de l'endoscope.

19. Système endoscopique selon la revendication 13, dans lequel les premier et deuxième mécanismes (13, 15, 88) d'engagement sont configurés pour connecter les première et deuxième parties (3, 8, 48, 50) de tubes de gainage et comprennent une disposition annulaire (43, 45) qui reçoit par encliquetage et maintient une bride circulaire (35) prévue sur l'une parmi les première et deuxième parties (3, 8, 48, 50) de tubes de gainage.

20. Système endoscopique selon la revendication 12, dans lequel le mécanisme (18) d'entraînement pouvant être actionné électriquement comprend une paire de rouleaux (25, 26).

21. Système endoscopique selon la revendication 12, comprenant une chambre de maintien (20) dans laquelle l'instrument de traitement est capable d'être stocké tout en étant retiré du canal (7) de l'endoscope (12, 70).

22. Système endoscopique selon la revendication 12, comprenant au moins une commande de commutation (27) destinée à actionner les éléments (5, 10) d'actionnement et le mécanisme d'entraînement destiné à déplacer la tête d'instrument de traitement de manière coulissante en va-et-vient à l'intérieur du canal (7).
